# EUROPEAN PATENT APPLICATION

(11) **EP 1 897 948 A1**
(43) Date of publication of application: **12.03.2008**
(21) Application number: 07017934.6
(22) Date of filing: 17.09.1999
(51) Int. Cl.: C12N 15/12, C07K 14/495, C07K 16/22, C07K 16/42

(54) **Mammalian transforming growth factor beta 9**

(30) Priority: 17.09.1998 US 154817
(62) Divisional of application: 99969114.0
(71) Applicant: ZymoGenetics, Inc., Seattle, WA 98102 (US)
(72) Inventor: Presnell, Scott R., Tacoma, WA 98102 (US); Taft, David W., Seattle, WA 98102 (US); Foley, Kevin P., Seattle, WA 98102 (US)
(74) Representative: Griffin, Philippa Jane

(57) **Abstract**

An isolated polynucleotide which encodes a polypeptide, wherein said polypeptide comprises an amino acid sequence that is at least 90% identical to an amino acid sequence selected from the group consisting of SEQ ID NO:2, SEQ ID NO:3, SEQ ID NO:4, SEQ ID NO:5, SEQ ID NO:9, SEQ ID NO:12, SEQ ID NO: 13, SEQ ID NO: 14, SEQ ID NO:15, SEQ ID NO:17, SEQ ID NO:18, SEQ ID NO: 19, SEQ ID NO: 20, SEQ ID NO: 21 and SEQ ID NO:22; the encoded polypeptide; and antibody that binds specifically thereto; and therapeutic uses thereof.

## Description

### BACKGROUND OF THE INVENTION

Proper control of the opposing processes of cell proliferation versus terminal differentiation and apoptotic programmed cell death is an important aspect of normal development and homeostasis, Raff, M.C., Cell, 86:173-175 (1996), and has been found to be altered in many human diseases. See, for example, Sawyers, C.L. et al., Cell, 64:337-350 (1991); Meyaard, L. et al., Science, 257:217-219 (1992); Guo, Q. et al., Nature Med., 4:957-962 (1998); Barinaga, M. Science, 273:735-737 (1996); Solary, E. et al., Eur. Respir. J., 9:1293-1305 (1996); Hamet, P. et al., J. Hypertension, 14:S65-S70, (1996); Roy, N. et al. Cell, 80:167-178 (1995); and Ambrosini, G., Nature Med., 8:917-921 (1997). Much progress has been made towards understanding the regulation of this balance. For example, signaling cascades have been elucidated through which extracellular stimuli, such as growth factors, peptide hormones, and cell-cell interactions control the commitment of precursor cells to specific lineages and their subsequent proliferative expansion, Morrison, S.J. et al., Cell, 88:287-298 (1997). Further, it has been found that cell cycle exit and terminal differentiation are coupled in most cell types. See, for example, Coppola, J.A. et al. Nature, 320:760-763 (1986); Freytag, S.O., Mol. Cell. Biol. 8:1614-1624 (1988); Lee, E.Y. et al., Genes Dev., 8:2008-2021 (1994); Morgenbesser, S.D., et al., Nature, 371:72-74 (1994); Casaccia-Bonnefil, P. et al., Genes Dev., 11:2335-2346 (1996); Zacksenhaus, E. et al., Genes Dev., 10:3051-3064 (1996); and Zhang, P. et al., Nature, 387:151-158 (1997). Apoptosis (programmed cell death) also plays an important role in many developmental and homeostatic processes, Raff, M.C., Nature, 356:397-400 (1992), and is often coordinately regulated with terminal differentiation, Jacobsen, K.A. et al., Blood, 84:2784-2794 (1994); Yan, Y. et al., Genes Dev., 11:973-983 (1997). Hence, it appears that the cell type of individual lineages, tissues, organs, or even entire multicellular organisms is the result of a finely tuned balance between increased cell production due to proliferation, and decreased numbers of cells resulting from terminal differentiation and apoptosis. This balance is most likely regulated coordinately by the convergence of multiple regulatory pathways. The identification of novel members of such networks can provide important insights into both normal cellular processes as well as the etiology and treatment of human disease states.

Interleukin 17 (IL-17) is a cytokine which has been implicated as an important regulator of the immune system, Spriggs, M.K., J. Clinical Immunology, 17:366-369 (1997), Broxmeyer, H.E., J. Experimental Medicine, 183:2411-2415 (1996), Yao, Z., et al., J. Immunology, 155:5483-5486(1995), Yao, Z., et al., Immunity, 3:811-821 (1995). Human IL-17 is almost exclusively produced by activated CD4+ memory T cells(however, in mice,CD4-/CD8- T cells also express IL-17), Aarvak, T., et al., J. Immunology, 162:1246-1251 (1999), Kennedy, J., et al., J. Interferon Cytokine Research,16:611-617 (1996). In contrast, the IL-17 receptor (IL-17R) appears to be ubiquitously expressed, Yao, Z., et al., Immunity, 3:811-821 (1995). IL-17 induces the secretion of IL-6, IL-8, monocyte chemotactic peptide-1 and G-CSF from a variety of different stromal cell types, but has no effect on cytokine production by lymphoid cells, Teunissen, M.B.M., J. Investigative Dermatology, 111:645-649 (1998), Jovanovic, D.V., et al., J. Immunology, 160:3513-3521 (1998), Chabaud, M., et al., J. Immunology, 161:409-414 (1998), Cai, X.-Y., et al., Immunology Letters, 62:51-58 (1998), Fossiez, F., et al., J. Experimental Medicine, 183:2593-2603 (1996). IL-17 also enhances the expression of ICAM-1 adhesion molecules on fibroblasts, and can stimulate granulopoiesis, Schwarzenberger P., et al., J. Immunology, 161:6383-9 (1998).. Taken together, these observations have suggested that IL-17 functions as a pro-inflammatory cytokine. IL-17 also promotes dendritic cell differentiation, osteoclastogenesis, can induce nitric oxide production in human osteoarthritis cartilage, and is present in synovial fluids from patients with rheumatoid arthritis, Antonysamy, M.A., et al., J. Immunology, 162:577-584 (1999), Kotake, S., et al., J. Clinical Investigation, 103:1345-1352, (1999), Attur, M.G., et al., Arthritis & Rheumatism, 40:1050-1053 (1997). Blocking IL-17 with a soluble IL-17R protein was found to suppress cardiac allograft rejection, which correlated with increased IL-17 mRNA in kidney biopsies from humans undergoing renal allograft rejection, Antonysamy, M.A., et al., J. Immunology, 162:577-584 (1999). Increased IL-17 mRNA expression is also observed in humans with multiple sclerosis, Matusevicius, D. et al., Multiple Sclerosis, 5:101-104 (1999). Further, IL-17 can promote tumorigenicity of human cervical tumors in nude mice, Tartour, E. et al., Cancer Res., 59:3698-36704 (1999). Hence, IL-17 appears to play an essential role in regulating the immune system and inflammatory processes.

Thus, there is a continuing need to discover new proteins involved with proliferation, differentiation, and apoptotic pathways. The in vivo activities of both inducers and inhibitors of these pathways illustrates the enormous clinical potential of, and need for, novel proliferation, differentiation, and apoptotic proteins, their agonists and antagonists. There is also a need to discover new agents which have anti-viral activity.

### SUMMARY OF THE INVENTION

The present invention addresses this need by providing a novel anti-viral polypeptide called transforming growth factor beta-9,hereinafter referred to as Ztgfβ-9, and related compositions and methods. This polypeptide has anti-viral activity as disclosed in Example 10 below. It may also be used to regulate the proliferation, differentiation and apoptosis of neurons glial cells, lymphocytes, hematopoietic cells and stromal cells.

Thus, one aspect of the present invention provides for an isolated Ztgfβ-9 polypeptide and polynucleotide. The human sequences are defined by SEQ ID NOs: 1 and 2.

The nucleotide sequence of SEQ ID NO:1 contains an open reading frame encoding a polypeptide of about 202 amino acids with the initial Met as shown in SEQ ID NO:1 and SEQ ID NO:2. A predicted signal sequence is comprised of amino acid residues 1, a methionine extending to and includes amino acid residue 15, an alanine. Thus a mature sequence excluding the signal sequence extends from amino acid residue 16, an alanine, to and including amino acid residue 202 a proline, of SEQ ID NO:2. This mature sequence is also represented by SEQ ID NO:3. In an alternative embodiment the signal sequence extends to and includes amino acid residue 16, an alanine. This produces a mature sequence which extends from amino acid 17, a glycine, to and including amino acid residue 202, a proline, of SEQ ID NO:2. This mature sequence is also represented by SEQ ID NO:4. In another alternative embodiment, the signal sequence extends to and includes amino acid residue 17, a glycine. This results in a mature sequence which extends from amino acid residue 18, an alanine, to and including amino acid residue 202, a proline, of SEQ ID NO:2. This mature sequence is further represented by SEQ ID NO:5. Another variant of Ztgfβ-9 is disclosed by SEQ ID NOs: 16 and 17. The mature sequence extends from amino acid residue 23, an alanine, to and including amino acid residue 209, a proline. The mature sequence is also defined by SEQ ID NO:18.

Murine Ztgfβ-9 is defined by SEQ ID NOs: 8 and 9. The signal sequence extends from the methionine at position 1 through the alanine at position 22. Thus the mature sequence extends from the alanine at position 23 of SEQ ID NO:9 through the arginine at position 205. The mature sequence is further represented by SEQ ID NO:12.

An additional embodiment of the present invention relates to a peptide or polypeptide which has the amino acid sequence of an epitope-bearing portion of a Ztgfβ-9 polypeptide having an amino acid sequence described above. Peptides or polypeptides having the amino acid sequence of an epitope-bearing portion of a Ztgfβ-9 polypeptide of the present invention include portions of such polypeptides with at least nine, preferably at least 15 and more preferably at least 30 to 50 amino acids, although epitope-bearing polypeptides of any length up to and including the entire amino acid sequence of a polypeptide of the present invention described above are also included in the present invention. Examples of such epitope-bearing polypeptides are SEQ ID NOs: 13, 14, 15, 19, 20, 21 and 22. Also claimed are any of these polypeptides that are fused to another polypeptide or carrier molecule. Also claimed is an isolated nucleic acid which encodes an epitope-bearing portion of a Ztgfβ-9 polypeptide.

The present invention is further comprised of an isolated peptide or polypeptide of the above-described peptides or polypeptides having an amino acid sequence modified by addition, deletion and/or replacement of one or more amino acid residues and which maintains the biological activity of said peptide or polypeptide.

Within a further aspect of the invention there is provided a chimeric polypeptide consisting essentially of a first portion and a second portion joined by a peptide bond. The first portion of the chimeric polypeptide consists essentially of (a) a Ztgfβ-9 polypeptide as described above (b) allelic variants of the polypeptides described above. The second portion of the chimeric polypeptide consists essentially of another polypeptide such as an affinity tag. Within one embodiment the affinity tag is an immunoglobulin F_{C} polypeptide. The invention also provides expression vectors encoding the chimeric polypeptides and host cells transfected to produce the chimeric polypeptides.

Another aspect of the present invention provides for isolated nucleic acid molecules comprising a polynucleotide selected from the group consisting of: (a) a nucleotide sequence encoding the Ztgfβ-9 polypeptides described above; and (b) a nucleotide sequence complementary to any of the nucleotide sequences in (a).

Further embodiments of the invention include isolated nucleic acid molecules that comprise a polynucleotide having a nucleotide sequence at least 90% homologous, and more preferably 95%, 97%, 98%, or 99% homologous to any of the nucleotide sequences in (a) or (b) above, or a polynucleotide which hybridizes under stringent hybridization conditions to a polynucleotide having a nucleotide sequence of (a)or (b) above.

Further embodiments of the invention include isolated polypeptides having an amino acid sequence that is at least 90% identical, and more preferably 95%, 97%, 98%, or 99% identical to any of the Ztgfβ-9 polypeptides and polynucleotides which encode these polypeptides.

Within another aspect of the invention there is provided an expression vector comprising (a) a transcription promoter; (b) a DNA segment encoding a polypeptide described above, and (c) a transcription terminator, wherein the promoter, DNA segment, and terminator are operably linked.

Within a third aspect of the invention there is provided a cultured eukaryotic cell into which has been introduced an expression vector as disclosed above, wherein said cell expresses a protein polypeptide encoded by the DNA segment.

In another embodiment of the present invention is an isolated antibody that binds specifically to a Ztgfβ-9 polypeptide described above. Also claimed is a method for producing antibodies which bind to a Ztgfβ-9 polypeptide comprising inoculating a mammal with a Ztgfβ-9 polypeptide or Ztgfβ-9 epitope-bearing polypeptide so that the mammal produces antibodies to the polypeptide; and isolating said antibodies.

These and other aspects of the invention will become evident upon reference to the following detailed description.

### DETAILED DESCRIPTION OF THE INVENTION

The teachings of all of the references cited herein are incorporated in their entirety herein by reference.

In the description that follows, a number of terms are used extensively. The following definitions are provided to facilitate understanding of the invention.

As used herein, "nucleic acid" or "nucleic acid molecule" refers to polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (*e.g.*, α-enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid molecule" also includes so-called "peptide nucleic acids," which comprise naturally-occurring or modified nucleic acid bases attached to a polyamide backbone. Nucleic acids can be either single stranded or double stranded.

The term "complement of a nucleic acid molecule" refers to a nucleic acid molecule having a complementary nucleotide sequence and reverse orientation as compared to a reference nucleotide sequence. For example, the sequence 5' ATGCACGGG 3' is complementary to 5' CCCGTGCAT 3'.

The term "contig" denotes a nucleic acid molecule that has a contiguous stretch of identical or complementary sequence to another nucleic acid molecule. Contiguous sequences are said to "overlap" a given stretch of a nucleic acid molecule either in their entirety or along a partial stretch of the nucleic acid molecule.

The term "degenerate nucleotide sequence" denotes a sequence of nucleotides that includes one or more degenerate codons as compared to a reference nucleic acid molecule that encodes a polypeptide. Degenerate codons contain different triplets of nucleotides, but encode the same amino acid residue (*i.e.*, GAU and GAC triplets each encode Asp).

The term "structural gene" refers to a nucleic acid molecule that is transcribed into messenger RNA (mRNA), which is then translated into a sequence of amino acids characteristic of a specific polypeptide.

An "isolated nucleic acid molecule" is a nucleic acid molecule that is not integrated in the genomic DNA of an organism. For example, a DNA molecule that encodes a growth factor that has been separated from the genomic DNA of a cell is an isolated DNA molecule. Another example of an isolated nucleic acid molecule is a chemically-synthesized nucleic acid molecule that is not integrated in the genome of an organism. A nucleic acid molecule that has been isolated from a particular species is smaller than the complete DNA molecule of a chromosome from that species.

A "nucleic acid molecule construct" is a nucleic acid molecule, either single- or double-stranded, that has been modified through human intervention to contain segments of nucleic acid combined and juxtaposed in an arrangement not existing in nature.

"Linear DNA" denotes non-circular DNA molecules having free 5' and 3' ends. Linear DNA can be prepared from closed circular DNA molecules, such as plasmids, by enzymatic digestion or physical disruption.

"Complementary DNA (cDNA)" is a single-stranded DNA molecule that is formed from an mRNA template by the enzyme reverse transcriptase. Typically, a primer complementary to portions of mRNA is employed for the initiation of reverse transcription. Those skilled in the art also use the term "cDNA" to refer to a double-stranded DNA molecule consisting of such a single-stranded DNA molecule and its complementary DNA strand. The term "cDNA" also refers to a clone of a cDNA molecule synthesized from an RNA template.

A "promoter" is a nucleotide sequence that directs the transcription of a structural gene. Typically, a promoter is located in the 5' non-coding region of a gene, proximal to the transcriptional start site of a structural gene. Sequence elements within promoters that function in the initiation of transcription are often characterized by consensus nucleotide sequences. These promoter elements include RNA polymerase binding sites, TATA sequences, CAAT sequences, differentiation-specific elements (DSEs; McGehee et al., Mol. Endocrinol. 7:551 (1993)), cyclic AMP response elements (CREs), serum response elements (SREs; Treisman, Seminars in Cancer Biol. 1:47 (1990)), glucocorticoid response elements (GREs), and binding sites for other transcription factors, such as CRE/ATF (O'Reilly et al., J. Biol. Chem. 267:19938 (1992)), AP2 (Ye et al., J. Biol. Chem. 269:25728 (1994)), SP1, cAMP response element binding protein (CREB; Loeken, Gene Expr. 3:253 (1993)) and octamer factors (see, in general, Watson et al., eds., Molecular Biology of the Gene, 4th ed. (The Benjamin/Cummings Publishing Company, Inc. 1987), and Lemaigre and Rousseau, Biochem. J. 303:1 (1994)). If a promoter is an inducible promoter, then the rate of transcription increases in response to an inducing agent. In contrast, the rate of transcription is not regulated by an inducing agent if the promoter is a constitutive promoter. Repressible promoters are also known.

A "core promoter" contains essential nucleotide sequences for promoter function, including the TATA box and start of transcription. By this definition, a core promoter may or may not have detectable activity in the absence of specific sequences that may enhance the activity or confer tissue specific activity.

A "regulatory element" is a nucleotide sequence that modulates the activity of a core promoter. For example, a regulatory element may contain a nucleotide sequence that binds with cellular factors enabling transcription exclusively or preferentially in particular cells, tissues, or organelles. These types of regulatory elements are normally associated with genes that are expressed in a "cell-specific," "tissue-specific," or "organelle-specific" manner. For example, the Ztgfβ-9 regulatory element preferentially induces gene expression in brain, spinal cord, heart, skeletal muscle, stomach, pancreas, adrenal gland, salivary gland, liver, small intestine, bone marrow, thymus, spleen, lymph node, heart, thyroid, trachea, testis, ovary and placenta.

An "enhancer" is a type of regulatory element that can increase the efficiency of transcription, regardless of the distance or orientation of the enhancer relative to the start site of transcription.

"Heterologous DNA" refers to a DNA molecule, or a population of DNA molecules, that does not exist naturally within a given host cell. DNA molecules heterologous to a particular host cell may contain DNA derived from the host cell species (*i.e.*, endogenous DNA) so long as that host DNA is combined with non-host DNA (*i.e.*, exogenous DNA). For example, a DNA molecule containing a non-host DNA segment encoding a polypeptide operably linked to a host DNA segment comprising a transcription promoter is considered to be a heterologous DNA molecule. Conversely, a heterologous DNA molecule can comprise an endogenous gene operably linked with an exogenous promoter. As another illustration, a DNA molecule comprising a gene derived from a wild-type cell is considered to be heterologous DNA if that DNA molecule is introduced into a mutant cell that lacks the wild-type gene.

A "polypeptide" is a polymer of amino acid residues joined by peptide bonds, whether produced naturally or synthetically. Polypeptides of less than about 10 amino acid residues are commonly referred to as "peptides."

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

A peptide or polypeptide encoded by a non-host DNA molecule is a "heterologous" peptide or polypeptide.

An "integrated genetic element" is a segment of DNA that has been incorporated into a chromosome of a host cell after that element is introduced into the cell through human manipulation. Within the present invention, integrated genetic elements are most commonly derived from linearized plasmids that are introduced into the cells by electroporation or other techniques. Integrated genetic elements are passed from the original host cell to its progeny.

A "cloning vector" is a nucleic acid molecule, such as a plasmid, cosmid, or bacteriophage, that has the capability of replicating autonomously in a host cell. Cloning vectors typically contain one or a small number of restriction endonuclease recognition sites that allow insertion of a nucleic acid molecule in a determinable fashion without loss of an essential biological function of the vector, as well as nucleotide sequences encoding a marker gene that is suitable for use in the identification and selection of cells transformed with the cloning vector. Marker genes typically include genes that provide tetracycline resistance or ampicillin resistance.

An "expression vector" is a nucleic acid molecule encoding a gene that is expressed in a host cell. Typically, an expression vector comprises a transcription promoter, a gene, and a transcription terminator. Gene expression is usually placed under the control of a promoter, and such a gene is said to be "operably linked to" the promoter. Similarly, a regulatory element and a core promoter are operably linked if the regulatory element modulates the activity of the core promoter.

A "recombinant host" is a cell that contains a heterologous nucleic acid molecule, such as a cloning vector or expression vector. In the present context, an example of a recombinant host is a cell that produces Ztgfβ-9 from an expression vector. In contrast, Ztgfβ-9 can be produced by a cell that is a "natural source" of Ztgfβ-9, and that lacks an expression vector.

"Integrative transformants" are recombinant host cells, in which heterologous DNA has become integrated into the genomic DNA of the cells.

A "fusion protein" is a hybrid protein expressed by a nucleic acid molecule comprising nucleotide sequences of at least two genes. For example, a fusion protein can comprise at least part of a Ztgfβ-9 polypeptide fused with a polypeptide that binds an affinity matrix. Such a fusion protein provides a means to isolate large quantities of Ztgfβ-9 using affinity chromatography.

The term "receptor" denotes a cell-associated protein that binds to a bioactive molecule termed a "ligand." This interaction mediates the effect of the ligand on the cell. Receptors can be membrane bound, cytosolic or nuclear; monomeric (*e.g.*, thyroid stimulating hormone receptor, beta-adrenergic receptor) or multimeric (*e.g.*, PDGF receptor, growth hormone receptor, IL-3 receptor, GM-CSF receptor, G-CSF receptor, erythropoietin receptor and IL-6 receptor). Membrane-bound receptors are characterized by a multi-domain structure comprising an extracellular ligand-binding domain and an intracellular effector domain that is typically involved in signal transduction. In certain membrane-bound receptors, the extracellular ligand-binding domain and the intracellular effector domain are located in separate polypeptides that comprise the complete functional receptor.

In general, the binding of ligand to receptor results in a conformational change in the receptor that causes an interaction between the effector domain and other molecule(s) in the cell, which in turn leads to an alteration in the metabolism of the cell. Metabolic events that are often linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids.

The term "secretory signal sequence" denotes a DNA sequence that encodes a peptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

An "isolated polypeptide" is a polypeptide that is essentially free from contaminating cellular components, such as carbohydrate, lipid, or other proteinaceous impurities associated with the polypeptide in nature. Typically, a preparation of isolated polypeptide contains the polypeptide in a highly purified form, *i.e.*, at least about 80% pure, at least about 90% pure, at least about 95% pure, greater than 95% pure, or greater than 99% pure. One way to show that a particular protein preparation contains an isolated polypeptide is by the appearance of a single band following sodium dodecyl sulfate (SDS)-polyacrylamide gel electrophoresis of the protein preparation and Coomassie Brilliant Blue staining of the gel. However, the term "isolated" does not exclude the presence of the same polypeptide in alternative physical forms, such as dimers or alternatively glycosylated or derivatized forms.

The terms "amino-terminal or N-terminal" and "carboxyl-terminal or C-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

The term "expression" refers to the biosynthesis of a gene product. For example, in the case of a structural gene, expression involves transcription of the structural gene into mRNA and the translation of mRNA into one or more polypeptides.

The term "splice variant" is used herein to denote alternative forms of RNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a polypeptide encoded by a splice variant of an mRNA transcribed from a gene.

As used herein, the term "immunomodulator" includes cytokines, stem cell growth factors, lymphotoxins, co-stimulatory molecules, hematopoietic factors, and synthetic analogs of these molecules.

The term "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like. Where subsequent dissociation of the complement/anti-complement pair is desirable, the complement/anti-complement pair preferably has a binding affinity of less than 10⁹ M⁻¹.

An "anti-idiotype antibody" is an antibody that binds with the variable region domain of an immunoglobulin. In the present context, an anti-idiotype antibody binds with the variable region of an anti- Ztgfβ-9 antibody, and thus, an anti-idiotype antibody mimics an epitope of Ztgfβ-9.

An "antibody fragment" is a portion of an antibody such as F(ab')₂, F(ab)_{2'} Fab', Fab, and the like. Regardless of structure, an antibody fragment binds with the same antigen that is recognized by the intact antibody. For example, a Ztgfβ-9 monoclonal antibody fragment binds with an epitope of Ztgfβ-9.

The term "antibody fragment" also includes a synthetic or a genetically engineered polypeptide that binds to a specific antigen, such as polypeptides consisting of the light chain variable region, "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

A "chimeric antibody" is a recombinant protein that contains the variable domains and complementary determining regions derived from a rodent antibody, while the remainder of the antibody molecule is derived from a human antibody.

"Humanized antibodies" are recombinant proteins in which murine complementarity determining regions of a monoclonal antibody have been transferred from heavy and light variable chains of the murine immunoglobulin into a human variable domain.

As used herein, a "therapeutic agent" is a molecule or atom which is conjugated to an antibody moiety to produce a conjugate which is useful for therapy. Examples of therapeutic agents include drugs, toxins, immunomodulators, chelators, boron compounds, photoactive agents or dyes, and radioisotopes.

A "detectable label" is a molecule or atom which can be conjugated to an antibody moiety to produce a molecule useful for diagnosis. Examples of detectable labels include chelators, photoactive agents, radioisotopes, fluorescent agents, paramagnetic ions, or other marker moieties.

The term "affinity tag" is used herein to denote a polypeptide segment that can be attached to a second polypeptide to provide for purification or detection of the second polypeptide or provide sites for attachment of the second polypeptide to a substrate. In principal, any peptide or protein for which an antibody or other specific binding agent is available can be used as an affinity tag. Affinity tags include a poly-histidine tract, protein A ' (Nilsson et al., EMBO J. 4:1075 (1985); Nilsson et al., Methods Enzymol. 198:3 (1991)), glutathione S transferase (Smith and Johnson, Gene 67:31 (1988)), Glu-Glu affinity tag (Grussenmeyer et al., Proc. Natl. Acad. Sci. USA 82:7952 (1985)), substance P, FLAG peptide (Hopp et al., Biotechnology 6:1204 (1988)), streptavidin binding peptide, or other antigenic epitope or binding domain. See, in general, Ford et al., Protein Expression and Purification 2:95 (1991). DNAs encoding affinity tags are available from commercial suppliers (*e.g*., Pharmacia Biotech, Piscataway, NJ).

A "naked antibody" is an entire antibody, as opposed to an antibody fragment, which is not conjugated with a therapeutic agent. Naked antibodies include both polyclonal and monoclonal antibodies, as well as certain recombinant antibodies, such as chimeric and humanized antibodies.

As used herein, the term "antibody component" includes both an entire antibody and an antibody fragment.

An "immunoconjugate" is a conjugate of an antibody component with a therapeutic agent or a detectable label.

As used herein, the term "antibody fusion protein" refers to a recombinant molecule that comprises an antibody component and a therapeutic agent. Examples of therapeutic agents suitable for such fusion proteins include immunomodulators ("antibody-immunomodulator fusion protein") and toxins ("antibody-toxin fusion protein").

A "tumor associated antigen" is a protein normally not expressed, or expressed at lower levels, by a normal counterpart cell. Examples of tumor associated antigens include alpha-fetoprotein, carcinoembryonic antigen, and Her-2/neu. Many other illustrations of tumor associated antigens are known to those of skill in the art. See, for example, Urban et al., Ann. Rev. Immunol. 10:617 (1992).

As used herein, an "infectious agent" denotes both microbes and parasites. A "microbe" includes viruses, bacteria, rickettsia, mycoplasma, protozoa, fungi and like microorganisms. A "parasite" denotes infectious, generally microscopic or very small multicellular invertebrates, or ova or juvenile forms thereof, which are susceptible to immune-mediated clearance or lytic or phagocytic destruction, such as malarial parasites, spirochetes, and the like.

An "infectious agent antigen" is an antigen associated with an infectious agent.

A "target polypeptide" or a "target peptide" is an amino acid sequence that comprises at least one epitope, and that is expressed on a target cell, such as a tumor cell, or a cell that carries an infectious agent antigen. T cells recognize peptide epitopes presented by a major histocompatibility complex molecule to a target polypeptide or target peptide and typically lyse the target cell or recruit other immune cells to the site of the target cell, thereby killing the target cell.

An "antigenic peptide" is a peptide which will bind a major histocompatibility complex molecule to form an MHC-peptide complex which is recognized by a T cell, thereby inducing a cytotoxic lymphocyte response upon presentation to the T cell. Thus, antigenic peptides are capable of binding to an appropriate major histocompatibility complex molecule and inducing a cytotoxic T cells response, such as cell lysis or specific cytokine release against the target cell which binds or expresses the antigen. The antigenic peptide can be bound in the context of a class I or class II major histocompatibility complex molecule, on an antigen presenting cell or on a target cell.

In eukaryotes, RNA polymerase II catalyzes the transcription of a structural gene to produce mRNA. A nucleic acid molecule can be designed to contain an RNA polymerase II template in which the RNA transcript has a sequence that is complementary to that of a specific mRNA. The RNA transcript is termed an "anti-sense RNA" and a nucleic acid molecule that encodes the anti-sense RNA is termed an "anti-sense gene." Anti-sense RNA molecules are capable of binding to mRNA molecules, resulting in an inhibition of mRNA translation or mRNA degradation.

An "anti-sense oligonucleotide specific for *Ztgfβ-9"* or a *"Ztgfβ-9* anti-sense oligonucleotide" is an oligonucleotide having a sequence (a) capable of forming a stable triplex with a portion of the *Ztgfβ-9* gene, or (b) capable of forming a stable duplex with a portion of an mRNA transcript of the *Ztgfβ-9* gene.

A "ribozyme" is a nucleic acid molecule that contains a catalytic center. The term includes RNA enzymes, self-splicing RNAs, self-cleaving RNAs, and nucleic acid molecules that perform these catalytic functions. A nucleic acid molecule that encodes a ribozyme is termed a "ribozyme gene."

An "external guide sequence" is a nucleic acid molecule that directs the endogenous ribozyme, RNase P, to a particular species of intracellular mRNA, resulting in the cleavage of the mRNA by RNase P. A nucleic acid molecule that encodes an external guide sequence is termed an "external guide sequence gene."

The term "variant human *Ztgfβ-9* gene" refers to nucleic acid molecules that encode a polypeptide having an amino acid sequence that is a modification of SEQ ID NO:2. Such variants include naturally-occurring polymorphisms of *Ztgfβ-9* genes, as well as synthetic genes that contain conservative amino acid substitutions of the amino acid sequence of SEQ ID NO:2. Additional variant forms of *Ztgfβ-9* genes are nucleic acid molecules that contain insertions or deletions of the nucleotide sequences described herein. A variant Ztgfβ-9 gene can be identified by determining whether the gene hybridizes with a nucleic acid molecule having the nucleotide sequence of SEQ ID NO:1, or its complement, under stringent conditions.

Similarly, the term "variant murine *Ztgfβ-9* gene" refers to nucleic acid molecules that encode a polypeptide having an amino acid sequence that is a modification of SEQ ID NO:9. A variant murine *Ztgfβ-9* gene can be identified by determining whether the gene hybridizes with a nucleic acid molecule having the nucleotide sequence of SEQ ID NO:8, or its complement, under stringent conditions.

Alternatively, variant *Ztgfβ-9* genes can be identified by sequence comparison. Two amino acid sequences have "100% amino acid sequence identity" if the amino acid residues of the two amino acid sequences are the same when aligned for maximal correspondence. Similarly, two nucleotide sequences have "100% nucleotide sequence identity" if the nucleotide residues of the two nucleotide sequences are the same when aligned for maximal correspondence. Sequence comparisons can be performed using standard software programs such as those included in the LASERGENE bioinformatics computing suite, which is produced by DNASTAR (Madison, Wisconsin). Other methods for comparing two nucleotide or amino acid sequences by determining optimal alignment are well-known to those of skill in the art (see, for example, Peruski and Peruski, The Internet and the New Biology: Tools for Genomic and Molecular Research (ASM Press, Inc. 1997), Wu et al. (eds.), "Information Superhighway and Computer Databases of Nucleic Acids and Proteins," in Methods in Gene Biotechnology, pages 123-151 (CRC Press, Inc. 1997), and Bishop (ed.), Guide to Human Genome Computing, 2nd Edition (Academic Press, Inc. 1998)). Particular methods for determining sequence identity are described below.

Regardless of the particular method used to identify a variant *Ztgfβ-9* gene or variant Ztgfβ-9 polypeptide, a variant gene or polypeptide encoded by a variant gene is functionally characterized by either its anti-viral or anti-proliferative activities, or by the ability to bind specifically to an anti-Ztgfβ-9 antibody.

The term "allelic variant" is used herein to denote any of two or more alternative forms of a gene occupying the same chromosomal locus. Allelic variation arises naturally through mutation, and may result in phenotypic polymorphism within populations. Gene mutations can be silent (no change in the encoded polypeptide) or may encode polypeptides having altered amino acid sequence. The term allelic variant is also used herein to denote a protein encoded by an allelic variant of a gene.

The term "ortholog" denotes a polypeptide or protein obtained from one species that is the functional counterpart of a polypeptide or protein from a different species. Sequence differences among orthologs are the result of speciation.

"Paralogs" are distinct but structurally related proteins made by an organism. Paralogs are believed to arise through gene duplication. For example, α-globin, β-globin, and myoglobin are paralogs of each other.

Due to the imprecision of standard analytical methods, molecular weights and lengths of polymers are understood to be approximate values. When such a value is expressed as "about" X or "approximately" X, the stated value of X will be understood to be accurate to ±10%.

Nucleic acid molecules encoding a human or mouse *Ztgfβ-9* gene can be obtained by screening a human or mouse cDNA or genomic library using polynucleotide probes based upon SEQ ID NO:1 or SEQ ID NO:8. These techniques are standard and well-established. As an illustration, a nucleic acid molecule that encodes a human *Ztgfβ-9* gene can be isolated from a human cDNA library. In this case, the first step would be to prepare the cDNA library by isolating RNA from brain, spinal cord, heart, skeletal muscle, stomach, pancreas, adrenal gland, salivary gland, liver, small intestine, bone marrow, thymus, spleen, lymph node, heart, thyroid, trachea, testis, ovary or placental tissue, using methods well-known to those of skill in the art. In general, RNA isolation techniques must provide a method for breaking cells, a means of inhibiting RNase-directed degradation of RNA, and a method of separating RNA from DNA, protein, and polysaccharide contaminants. For example, total RNA can be isolated by freezing tissue in liquid nitrogen, grinding the frozen tissue with a mortar and pestle to lyse the cells, extracting the ground tissue with a solution of phenol/chloroform to remove proteins, and separating RNA from the remaining impurities by selective precipitation with lithium chloride (see, for example, Ausubel et al. (eds.), Short Protocols in Molecular Biology, 3rd Edition, pages 4-1 to 4-6 (John Wiley & Sons 1995) ["Ausubel (1995)"]; Wu et al., Methods in Gene Biotechnology, pages 33-41 (CRC Press, Inc. 1997) ["Wu (1997)"]).

Alternatively, total RNA can be isolated from brain or spinal cord tissue as well as heart, skeletal muscle, stomach, pancreas, adrenal gland, salivary gland, liver, small intestine, bone marrow, thymus, spleen, lymph node, thyroid, trachea, testis, ovary or placenta by extracting ground tissue with guanidinium isothiocyanate, extracting with organic solvents, and separating RNA from contaminants using differential centrifugation (see, for example, Chirgwin et al., Biochemistry 18:52 (1979); Ausubel (1995) at pages 4-1 to 4-6; Wu (1997) at pages 33-41). To construct a cDNA library, poly(A)⁺ RNA must be isolated from a total RNA preparation. Poly(A)⁺ RNA can be isolated from total RNA using the standard technique of oligo(dT)-cellulose chromatography (see, for example, Aviv and Leder, Proc. Nat'l Acad. Sci. USA 69:1408 (1972); Ausubel (1995) at pages 4-11 to 4-12). Double-stranded cDNA molecules are synthesized from poly (A)⁺ RNA using techniques well-known to those in the art. (see, for example, Wu (1997) at pages 41-46). Moreover, commercially available kits can be used to synthesize double-stranded cDNA molecules. For example, such kits are available from Life Technologies, Inc. (Gaithersburg, MD), CLONTECH Laboratories, Inc. (Palo Alto, CA), Promega Corporation (Madison, WI) and STRATAGENE (La Jolla, CA).

Various cloning vectors are appropriate for the construction of a cDNA library. For example, a cDNA library can be prepared in a vector derived from bacteriophage, such as a λgt10 vector. See, for example, Huynh et al., "Constructing and Screening cDNA Libraries in λgt10 and λgt11," in DNA Cloning: A Practical Approach Vol. I, Glover (ed.), page 49 (IRL Press, 1985); Wu (1997) at pages 47-52. Alternatively, double-stranded cDNA molecules can be inserted into a plasmid vector, such as a pBLUESCRIPT vector (STRATAGENE; La Jolla, CA), a LAMDAGEM-4 (Promega Corp.) or other commercially available vectors. Suitable cloning vectors also can be obtained from the American Type Culture Collection (Manassas, VA). To amplify the cloned cDNA molecules, the cDNA library is inserted into a prokaryotic host, using standard techniques. For example, a cDNA library can be introduced into competent *E. coli* DH5 cells, which can be obtained, for example, from Life Technologies, Inc. (Gaithersburg, MD).

A human genomic library can be prepared by means well-known in the art (see, for example, Ausubel (1995) at pages 5-1 to 5-6; Wu (1997) at pages 307-327). Genomic DNA can be isolated by lysing tissue with the detergent Sarkosyl, digesting the lysate with proteinase K, clearing insoluble debris from the lysate by centrifugation, precipitating nucleic acid from the lysate using isopropanol, and purifying resuspended DNA on a cesium chloride density gradient. DNA fragments that are suitable for the production of a genomic library can be obtained by the random shearing of genomic DNA or by the partial digestion of genomic DNA with restriction endonucleases. Genomic DNA fragments can be inserted into a vector, such as a bacteriophage or cosmid vector, in accordance with conventional techniques, such as the use of restriction enzyme digestion to provide appropriate termini, the use of alkaline phosphatase treatment to avoid undesirable joining of DNA molecules, and ligation with appropriate ligases. Techniques for such manipulation are well-known in the art (see, for example, Ausubel (1995) at pages 5-1 to 5-6; Wu (1997) at pages 307-327).

Nucleic acid molecules that encode a human *Ztgfβ-9* gene can also be obtained using the polymerase chain reaction (PCR) with oligonucleotide primers having nucleotide sequences that are based upon the nucleotide sequences of the human *Ztgfβ-9* gene, as described herein. General methods for screening libraries with PCR are provided by, for example, Yu et al., "Use of the Polymerase Chain Reaction to Screen Phage Libraries," in Methods in Molecular Biology, Vol. 15: PCR Protocols: Current Methods and Applications, White (ed.), pages 211-215 (Humana Press, Inc. 1993). Moreover, techniques for using PCR to isolate related genes are described by, for example, Preston, "Use of Degenerate Oligonucleotide Primers and the Polymerase Chain Reaction to Clone Gene Family Members," in Methods in Molecular Biology, Vol. 15: PCR Protocols: Current Methods and Applications, White (ed.), pages 317-337 (Humana Press, Inc. 1993). Alternatively, human genomic libraries can be obtained from commercial sources such as Research Genetics (Huntsville, AL) and the American Type Culture Collection (Manassas, VA). A library containing cDNA or genomic clones can be screened with one or more polynucleotide probes based upon SEQ ID NO:1, using standard methods (see, for example, Ausubel (1995) at pages 6-1 to 6-11).

Anti- Ztgfβ-9 antibodies, produced as described below, can also be used to isolate DNA sequences that encode human *Ztgfβ-9* genes from cDNA libraries. For example, the antibodies can be used to screen λgt11 expression libraries, or the antibodies can be used for immunoscreening following hybrid selection and translation (see, for example, Ausubel (1995) at pages 6-12 to 6-16; Margolis et al., "Screening λ expression libraries with antibody and protein probes," in DNA Cloning 2: Expression Systems, 2nd Edition, Glover et al. (eds.), pages 1-14 (Oxford University Press 1995)).

As an alternative, an *Ztgfβ-9* gene can be obtained by synthesizing nucleic acid molecules using mutually priming long oligonucleotides and the nucleotide sequences described herein (see, for example, Ausubel (1995) at pages 8-8 to 8-9). Established techniques using the polymerase chain reaction provide the ability to synthesize DNA molecules at least two kilobases in length (Adang et al., Plant Molec. Biol. 21:1131 (1993), Bambot et al., PCR Methods and Applications 2:266 (1993), Dillon et al., "Use of the Polymerase Chain Reaction for the Rapid Construction of Synthetic Genes," in Methods in Molecular Biology, Vol. 15: PCR Protocols: Current Methods and Applications, White (ed.), pages 263-268, (Humana Press, Inc. 1993), and Holowachuk et al., PCR Methods Appl. 4:299 (1995)). The sequence of an *Ztgf*β-9 cDNA or *Ztgfβ-9* genomic fragment can be determined using standard methods. Moreover, the identification of genomic fragments containing an *Ztgfβ-9* promoter or regulatory element can be achieved using well-established techniques, such as deletion analysis (see, generally, Ausubel (1995)).

Cloning of 5' flanking sequences also facilitates production of Ztgfβ-9 proteins by "gene activation," following the methods disclosed in U.S. Patent No. 5,641,670. Briefly, expression of an endogenous *Ztgfβ-9* gene in a cell is altered by introducing into the *Ztgfβ-9* locus a DNA construct comprising at least a targeting sequence, a regulatory sequence, an exon, and an unpaired splice donor site. The targeting sequence is a *Ztgfβ-9* 5' non-coding sequence that permits homologous recombination of the construct with the endogenous *Ztgfβ-9* locus, whereby the sequences within the construct become operably linked with the endogenous *Ztgf*β-*9* coding sequence. In this way, an endogenous *Ztgf*β-*9* promoter can be replaced or supplemented with other regulatory sequences to provide enhanced, tissue-specific, or otherwise regulated expression.

Additionally, the polynucleotides of the present invention can be synthesized using a DNA synthesizer. Currently the method of choice is the phosphoramidite method. If chemically synthesized double stranded DNA is required for an application such as the synthesis of a gene or a gene fragment, then each complementary strand is made separately. The production of short genes (60 to 80 bp) is technically straightforward and can be accomplished by synthesizing the complementary strands and then annealing them. For the production of longer genes (>300 bp), however, special strategies must be invoked, because the coupling efficiency of each cycle during chemical DNA synthesis is seldom 100%. To overcome this problem, synthetic genes (double-stranded) are assembled in modular form from single-stranded fragments that are from 20 to 100 nucleotides in length. In addition to the protein coding sequence, synthetic genes can be designed with terminal sequences that facilitate insertion into a restriction endonuclease sites of a cloning vector and other sequences should also be added that contain signals for the proper initiation and termination of transcription and translation. See Glick, Bernard R. and Jack J. Pasternak, Molecular Biotechnology, Principles & Applications of Recombinant DNA, (ASM Press, Washington, D.C. 1994), Itakura, K. et al. Synthesis and use of synthetic oligonucleotides. Annu. Rev. Biochem. 53 : 323-356 (1984), and Climie, S. et al. Chemical synthesis of the thymidylate synthase gene. Proc. Natl. Acad. Sci. USA 87 :633-637 (1990).

Within preferred embodiments of the invention the isolated polynucleotides will hybridize to similar sized regions of the DNA of SEQ ID NO:1, or a sequence complementary thereto, under stringent conditions. In general, stringent conditions are selected to be about 5°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Typical stringent conditions are those in which the salt concentration is about 0.02 M or less at pH 7 and the temperature is at least about 60°C. As previously noted, the isolated polynucleotides of the present invention include DNA and RNA. Methods for isolating DNA and RNA are well known in the art. Total RNA can be prepared using guanidine HCl extraction followed by isolation by centrifugation in a CsCl gradient [Chirgwin et al., Biochemistry 18:52-94 (1979)]. Poly (A)⁺ RNA is prepared from total RNA using the method of Aviv and Leder, Proc. Natl. Acad. Sci. USA 69:1408-1412 (1972). Complementary DNA (cDNA) is prepared from poly(A)⁺ RNA using known methods. Polynucleotides encoding Ztgfβ-9 polypeptides are then identified and isolated by, for example, hybridization or PCR.

Those skilled in the art will recognize that the sequences disclosed in SEQ ID NOS:1 and 2 represent a single allele of the human. There are a number of naturally occurring mature N-terminal variants having the leader sequence cleaved at differing positions. Allelic variants of these sequences can be cloned by probing cDNA or genomic libraries from different individuals according to standard procedures. The present invention further provides counterpart proteins and polynucleotides from other species ("species orthologs"). Of particular interest are Ztgfβ-9 polypeptides from other mammalian species, including murine, porcine, ovine, bovine, canine, feline, equine, and other primates. Species orthologs of the human Ztgfβ-9 protein can be cloned using information and compositions provided by the present invention in combination with conventional cloning techniques. For example, a cDNA can be cloned using mRNA obtained from a tissue or cell type that expresses the gene. Suitable sources of mRNA can be identified by probing Northern blots with probes designed from the sequences disclosed herein. A library is then prepared from mRNA of a positive tissue or cell line. A protein-encoding cDNA can then be isolated by a variety of methods, such as by probing with a complete or partial human cDNA or with one or more sets of degenerate probes based on the disclosed sequences. A cDNA can also be cloned using the polymerase chain reaction, or PCR (Mullis, U.S. Patent No. 4,683,202), using primers designed from the sequences disclosed herein. Within an additional method, the cDNA library can be used to transform or transfect host cells, and expression of the cDNA of interest can be detected with an antibody to the protein. Similar techniques can also be applied to the isolation of genomic clones. As used and claimed the language "an isolated polynucleotide which encodes a polypeptide, said polynucleotide being defined by SEQ ID NO: 2" includes all allelic variants and species orthologs of the polypeptide of SEQ ID NOs:2, 3, 4 and 5.

Within preferred embodiments of the invention, isolated nucleic acid molecules that encode human Ztgfβ-9 can hybridize to nucleic acid molecules having the nucleotide sequence of SEQ ID NO:1, or a sequence complementary thereto, under "stringent conditions." In general, stringent conditions are selected to be about 5°C lower than the thermal melting point (Tₘ) for the specific sequence at a defined ionic strength and pH. The Tₘ is the temperature (under defined ionic strength and pH) at which 50% of the target sequence hybridizes to a perfectly matched probe.

As an illustration, a nucleic acid molecule encoding a variant Ztgfβ-9 polypeptide can be hybridized with a nucleic acid molecule having the nucleotide sequence of SEQ ID NO:1 (or its complement) at 42°C overnight in a solution comprising 50% formamide, 5xSSC (1xSSC: 0.15 M sodium chloride and 15 mM sodium citrate), 50 mM sodium phosphate (pH 7.6), 5x Denhardt's solution (100x Denhardt's solution: 2% (w/v) Ficoll 400, 2% (w/v) polyvinylpyrrolidone, and 2% (w/v) bovine serum albumin), 10% dextran sulfate, and 20 µg/ml denatured, sheared salmon sperm DNA. One of skill in the art can devise variations of these hybridization conditions. For example, the hybridization mixture can be incubated at a higher temperature, such as about 65°C, in a solution that does not contain formamide. Moreover, premixed hybridization solutions are available (*e.g.*, EXPRESSHYB Hybridization Solution from CLONTECH Laboratories, Inc.), and hybridization can be performed according to the manufacturer's instructions. Following hybridization, the nucleic acid molecules can be washed to remove non-hybridized nucleic acid molecules under stringent conditions, or under highly stringent conditions. Typical stringent washing conditions include washing in a solution of 0.5x - 2x SSC with 0.1% sodium dodecyl sulfate (SDS) at 55 - 65°C. That is, nucleic acid molecules encoding a variant Ztgfβ-9 polypeptide hybridize with a nucleic acid molecule having the nucleotide sequence of SEQ ID NO:1 (or its complement) under stringent washing conditions, in which the wash stringency is equivalent to 0.5x - 2x SSC with 0.1% SDS at 55 - 65°C, including 0.5x SSC with 0.1% SDS at 55°C, or 2xSSC with 0.1% SDS at 65°C. One of skill in the art can readily devise equivalent conditions, for example, by substituting SSPE for SSC in the wash solution.

Typical highly stringent washing conditions include washing in a solution of 0.1x - 0.2x SSC with 0.1% sodium dodecyl sulfate (SDS) at 50 - 65°C. In other words, nucleic acid molecules encoding a variant Ztgfβ-9 polypeptide hybridize with a nucleic acid molecule having the nucleotide sequence of SEQ ID NO:1 (or its complement) under highly stringent washing conditions, in which the wash stringency is equivalent to 0.1x - 0.2x SSC with 0.1% SDS at 50 - 65°C, including 0.1x SSC with 0.1% SDS at 50°C, or 0.2xSSC with 0.1% SDS at 65°C.

The present invention also provides isolated Ztgfβ-9 polypeptides that have a substantially similar sequence identity to the polypeptides of SEQ ID NOs:2,3, 4, 5, 9, 12, 17, 18 or their orthologs. The term "substantially similar sequence identity" is used herein to denote polypeptides having at least 70%, at least 80%, at least 90%, at least 95% or greater than 95% and 99% sequence identity to the sequences shown in SEQ ID NOs:2, 3, 4, 5, 9, 12, 17, 18 or their orthologs.

The present invention also contemplates *Ztgfβ-9* variant nucleic acid molecules that can be identified using two criteria: a determination of the similarity between the encoded polypeptide with the amino acid sequence of SEQ ID NOs:2, 3, 4, 5, 9, 12, 17 or 18, and a hybridization assay, as described above. Such *Ztgfβ-9* variants include nucleic acid molecules (1) that hybridize with a nucleic acid molecule having the nucleotide sequence of SEQ ID NO:1, SEQ ID NO: 9 or SEQ ID NO:16 (or their complement) under stringent washing conditions, in which the wash stringency is equivalent to 0.5x - 2x SSC with 0.1% SDS at 55 - 65°C, and (2) that encode a polypeptide having at least 70%, at least 80%, at least 90%, at least 95% or greater than 95% sequence identity to the amino acid sequence of SEQ ID NOs:2, 3, 4, 5, 9, 12, 17 or 18. Alternatively, *Ztgfβ-9* variants can be characterized as nucleic acid molecules (1) that hybridize with a nucleic acid molecule having the nucleotide sequence of SEQ ID NO:1 (or its complement) under highly stringent washing conditions, in which the wash stringency is equivalent to 0.1x - 0.2x SSC with 0.1% SDS at 50 - 65°C, and (2) that encode a polypeptide having at least 70%, at least 80%, at least 90%, at least 95% or greater than 95% or 99% sequence identity to the amino acid sequence of SEQ ID NOs:2, 3, 4, 5, 9, 12, 17 or 18.

The present invention also contemplates human *ztgf*β*-9* variant nucleic acid molecules identified by at least one of hybridization analysis and sequence identity determination, with reference to SEQ ID NO:2. The present invention further includes murine *Ztgfβ-9* variant nucleic acid molecules identified by at least one of hybridization analysis and sequence identity determination, with reference to SEQ ID NOs:8 and 9. For example, using the approach discussed above, murine Ztgfβ-9 variant nucleic acid molecules can be identified using at least one of three criteria: (1) hybridization with a nucleic acid molecule having the nucleotide sequence of SEQ ID NO:8(or its complement) under stringent washing conditions, in which the wash stringency is equivalent to 0.5x - 2x SSC with 0.1% SDS at 55 - 65°C, (2) hybridization with a nucleic acid molecule having the nucleotide sequence of SEQ ID NO:8 (or its complement) under highly stringent washing conditions, in which the wash stringency is equivalent to 0.1x - 0.2x SSC with 0.1% SDS at 50 - 65°C, and (3) an amino acid percent identity that is at least 70%, at least 80%, at least 90%, at least 95% or greater than 95% sequence identity to the amino acid sequence of SEQ ID NO:12.

Percent sequence identity is determined by conventional methods. See, for example, Altschul et al., Bull. Math. Bio. 48:603 (1986), and Henikoff and Henikoff, Proc. Natl. Acad. Sci. USA 89:10915 (1992). Briefly, two amino acid sequences are aligned to optimize the alignment scores using a gap opening penalty of 10, a gap extension penalty of 1, and the "BLOSUM 62" scoring matrix of Henikoff and Henikoff (ibid.) as shown in Table 1 (amino acids are indicated by the standard one-letter codes). The percent identity is then calculated as: ([Total number of identical matches]/ [length of the longer sequence plus the number of gaps introduced into the longer sequence in order to align the two sequences])(100).

**Table 1**

| | A | R | N | D | C | Q | E | G | H | I | L | K | M | F | P | S | T | W | Y | V |
|---|---|---|---|---|---|---|---|---|---|---|---|---|---|---|---|---|---|---|---|---|
| A | 4 | | | | | | | | | | | | | | | | | | | |
| R | -1 | 5 | | | | | | | | | | | | | | | | | | |
| N | -2 | 0 | 6 | | | | | | | | | | | | | | | | | |
| D | -2 | -2 | 1 | 6 | | | | | | | | | | | | | | | | |
| C | 0 | -3 | -3 | -3 | 9 | | | | | | | | | | | | | | | |
| Q | -1 | 1 | 0 | 0 | -3 | 5 | | | | | | | | | | | | | | |
| E | -1 | 0 | 0 | 2 | -4 | 2 | 5 | | | | | | | | | | | | | |
| G | 0 | -2 | 0 | -1 | -3 | -2 | -2 | 6 | | | | | | | | | | | | |
| H | -2 | 0 | 1 | -1 | -3 | 0 | 0 | -2 | 8 | | | | | | | | | | | |
| I | -1 | -3 | -3 | -3 | -1 | -3 | -3 | -4 | -3 | 4 | | | | | | | | | | |
| L | -1 | -2 | -3 | -4 | -1 | -2 | -3 | -4 | -3 | 2 | 4 | | | | | | | | | |
| K | -1 | 2 | 0 | -1 | -3 | 1 | 1 | -2 | -1 | -3 | -2 | 5 | | | | | | | | |
| M | -1 | -1 | -2 | -3 | -1 | 0 | -2 | -3 | -2 | 1 | 2 | -1 | 5 | | | | | | | |
| F | -2 | -3 | -3 | -3 | -2 | -3 | -3 | -3 | -1 | 0 | 0 | -3 | 0 | 6 | | | | | | |
| P | -1 | -2 | -2 | -1 | -3 | -1 | -1 | -2 | -2 | -3 | -3 | -1 | -2 | -4 | 7 | | | | | |
| S | 1 | -1 | 1 | 0 | -1 | 0 | 0 | 0 | -1 | -2 | -2 | 0 | -1 | -2 | -1 | 4 | | | | |
| T | 0 | -1 | 0 | -1 | -1 | -1 | -1 | -2 | -2 | -1 | -1 | -1 | -1 | -2 | -1 | 1 | 5 | | | |
| W | -3 | -3 | -4 | -4 | -2 | -2 | -3 | -2 | -2 | -3 | -2 | -3 | -1 | 1 | -4 | -3 | -2 | 11 | 7 | |
| Y | --2 | --2 | -2 | -3 | -2 | -1 | -2 | -3 | 2 | -1 | -1 | -2 | -1 | 3 | -3 | -2 | -2 | 2 | 7 | |
| V | 0 | -3 | -3 | -3 | -1 | -2 | -2 | -3 | -3 | 3 | 1 | -2 | 1 | -1 | -2 | -2 | 0 | -3 | -1 | 4 |

Those skilled in the art appreciate that there are many established algorithms available to align two amino acid sequences. The "FASTA" similarity search algorithm of Pearson and Lipman is a suitable protein alignment method for examining the level of identity shared by an amino acid sequence disclosed herein and the amino acid sequence of a putative Ztgfβ-9 variant. The FASTA algorithm is described by Pearson and Lipman, Proc. Nat'1 Acad. Sci. USA 85:2444 (1988), and by Pearson, Meth. Enzymol. 183:63 (1990). Briefly, FASTA first characterizes sequence similarity by identifying regions shared by the query sequence (*e.g.*, SEQ ID NO:2) and a test sequence that have either the highest density of identities (if the ktup variable is 1) or pairs of identities (if ktup=2), without considering conservative amino acid substitutions, insertions, or deletions. The ten regions with the highest density of identities are then re-scored by comparing the similarity of all paired amino acids using an amino acid substitution matrix, and the ends of the regions are "trimmed" to include only those residues that contribute to the highest score. If there are several regions with scores greater than the "cutoff" value (calculated by a predetermined formula based upon the length of the sequence and the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm (Needleman and Wunsch, J. Mol. Biol. 48:444 (1970); Sellers, SIAM J. Appl. Math. 26:787 (1974)), which allows for amino acid insertions and deletions. Illustrative parameters for FASTA analysis are: ktup=1, gap opening penalty=10, gap extension penalty=1, and substitution matrix=BLOSUM62. These parameters can be introduced into a FASTA program by modifying the scoring matrix file ("SMATRIX"), as explained in Appendix 2 of Pearson, Meth. Enzymol. 183:63 (1990).

FASTA can also be used to determine the sequence identity of nucleic acid molecules using a ratio as disclosed above. For nucleotide sequence comparisons, the ktup value can range between one to six, preferably from three to six, most preferably three, with other parameters set as described above.

The present invention includes nucleic acid molecules that encode a polypeptide having a conservative amino acid change, compared with the amino acid sequence of SEQ ID NO:2, SEQ ID NO:3, SEQ ID NO: 4, SEQ ID NO:5, SEQ ID NO:9, SEQ ID NO:12, SEQ ID NO:17 or SEQ ID NO:18. That is, variants can be obtained that contain one or more amino acid substitutions of SEQ ID NO:2, SEQ ID NO:3, SEQ ID NO: 4 SEQ ID NO:5 SEQ ID NO:9 or SEQ ID NO:12, in which an alkyl amino acid is substituted for an alkyl amino acid in a Ztgfβ-9 amino acid sequence, an aromatic amino acid is substituted for an aromatic amino acid in an Ztgfβ-9 amino acid sequence, a sulfur-containing amino acid is substituted for a sulfur-containing amino acid in an Ztgfβ-9 amino acid sequence, a hydroxy-containing amino acid is substituted for a hydroxy-containing amino acid in a Ztgfβ-9 amino acid sequence, an acidic amino acid is substituted for an acidic amino acid in a Ztgfβ-9 amino acid sequence, a basic amino acid is substituted for a basic amino acid in a Ztgfβ-9 amino acid sequence, or a dibasic monocarboxylic amino acid is substituted for a dibasic monocarboxylic amino acid in an Ztgfβ-9 amino acid sequence.

Among the common amino acids, for example, a "conservative amino acid substitution" is illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine. For example, variant Ztgfβ-9 polypeptides that have an amino acid sequence that differs from either SEQ ID NOs:2, 3, 4, 5 or 12 can be obtained by substituting a threonine residue for Ser, by substituting a valine residue for Ile, by substituting an aspartate residue for Glu, or by substituting a valine residue for Ile. Additional variants can be obtained by producing polypeptides having two or more of these amino acid substitutions.

Variants of either the human or the murine Ztgfβ-9 can be devised by aligning the amino acid sequences of SEQ ID NO:3 and SEQ ID NO:12, and by noting any differences in the corresponding amino acid residues.

The BLOSUM62 table is an amino acid substitution matrix derived from about 2,000 local multiple alignments of protein sequence segments, representing highly conserved regions of more than 500 groups of related proteins (Henikoff and Henikoff, Proc. Nat'1 Acad. Sci. USA 89:10915 (1992)). Accordingly, the BLOSUM62 substitution frequencies can be used to define conservative amino acid substitutions that may be introduced into the amino acid sequences of the present invention. Although it is possible to design amino acid substitutions based solely upon chemical properties (as discussed above), the language "conservative amino acid substitution" preferably refers to a substitution represented by a BLOSUM62 value of greater than -1. For example, an amino acid substitution is conservative if the substitution is characterized by a BLOSUM62 value of 0, 1, 2, or 3. According to this system, preferred conservative amino acid substitutions are characterized by a BLOSUM62 value of at least 1 (*e.g.,* 1, 2 or 3), while more preferred conservative amino acid substitutions are characterized by a BLOSUM62 value of at least 2 (e.g., 2 or 3) .

Particular variants of human or murine Ztgfβ-9 are characterized by having at least 70%, at least 80%, at least 90%, at least 95% or 99% or greater sequence identity to the corresponding human (i.e., SEQ ID NOs: 2, 3, 4, 5 or 17) or murine (i.e., SEQ ID NOs:9 or 12) amino acid sequences, wherein the variation in amino acid sequence is due to one or more conservative amino acid substitutions.

Conservative amino acid changes in a *Ztgf*β*-9* gene can be introduced by substituting nucleotides for the nucleotides recited in any one of SEQ ID NOs:1 or 9. Such "conservative amino acid" variants can be obtained, for example, by oligonucleotide-directed mutagenesis, linker-scanning mutagenesis, mutagenesis using the polymerase chain reaction, and the like (see Ausubel (1995) at pages 8-10 to 8-22; and McPherson (ed.), Directed Mutagenesis: A Practical Approach (IRL Press 1991)). The ability of such variants to promote anti-viral or anti-proliferative activity can be determined using a standard method, such as the assay described herein. Alternatively, a variant Ztgfβ-9 polypeptide can be identified by the ability to specifically bind anti- Ztgfβ-9 antibodies.

The proteins of the present invention can also comprise non-naturally occurring amino acid residues. Non-naturally occurring amino acids include, without limitation, trans-3-methylproline, 2,4-methanoproline, *cis*-4-hydroxyproline, *trans*-4-hydroxyproline, *N-*methylglycine, *allo*-threonine, methylthreonine, hydroxyethylcysteine, hydroxyethylhomocysteine, nitroglutamine, homoglutamine, pipecolic acid, thiazolidine carboxylic acid, dehydroproline, 3- and 4-methylproline, 3,3-dimethylproline, tert-leucine, norvaline, 2-azaphenylalanine, 3-azaphenylalanine, 4-azaphenylalanine, and 4-fluorophenylalanine. Several methods are known in the art for incorporating non-naturally occurring amino acid residues into proteins. For example, an in vitro system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is typically carried out in a cell-free system comprising an *E. coli* S30 extract and commercially available enzymes and other reagents. Proteins are purified by chromatography. See, for example, Robertson et al., J. Am. Chem. Soc. 113:2722 (1991), Ellman et al., Methods Enzymol. 202:301 (1991), Chung et al., Science 259:806 (1993), and Chung et al., Proc. Nat'l Acad. Sci. USA 90:10145 (1993) .

In a second method, translation is carried out in *Xenopus* oocytes by microinjection of mutated mRNA and chemically aminoacylated suppressor tRNAs (Turcatti et al., J. Biol. Chem. 271:19991 (1996)). Within a third method, *E. coli* cells are cultured in the absence of a natural amino acid that is to be replaced (e.g., phenylalanine) and in the presence of the desired non-naturally occurring amino acid(s) (*e.g.*, 2azaphenylalanine, 3-azaphenylalanine, 4-azaphenylalanine, or 4-fluorophenylalanine). The non-naturally occurring amino acid is incorporated into the protein in place of its natural counterpart. See, Koide et al., Biochem. 33:7470 (1994). Naturally occurring amino acid residues can be converted to non-naturally occurring species by in vitro chemical modification. Chemical modification can be combined with site-directed mutagenesis to further expand the range of substitutions (Wynn and Richards, Protein Sci. 2:395 (1993)).

A limited number of non-conservative amino acids, amino acids that are not encoded by the genetic code, non-naturally occurring amino acids, and unnatural amino acids may be substituted for Ztgfβ-9 amino acid residues.

Essential amino acids in the polypeptides of the present invention can be identified according to procedures known in the art, such as site-directed mutagenesis or alanine-scanning mutagenesis (Cunningham and Wells, Science 244:1081 (1989), Bass et al., Proc. Nat'1 Acad. Sci. USA 88:4498 (1991), Coombs and Corey, "Site-Directed Mutagenesis and Protein Engineering," in Proteins: Analysis and Design, Angeletti (ed.), pages 259-311 (Academic Press, Inc. 1998)). In the latter technique, single alanine mutations are introduced at every residue in the molecule, and the resultant mutant molecules are tested for biological activity as disclosed below to identify amino acid residues that are critical to the activity of the molecule. See also, Hilton et al., J. Biol. Chem. 271:4699 (1996).

**Table 2**

| Conservative amino acid substitutions | |
|---|---|
| Basic: | arginine |
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

Essential amino acids in the polypeptides of the present invention can be identified according to procedures known in the art, such as site-directed mutagenesis or alanine-scanning mutagenesis [Cunningham and Wells, Science 244: 1081-1085 (1989); Bass et al., Proc. Natl. Acad. Sci. USA 88:4498-4502 (1991)]. In the latter technique, single alanine mutations are introduced at every residue in the molecule, and the resultant mutant molecules are tested for biological activity (e.g., ligand binding and signal transduction) to identify amino acid residues that are critical to the activity of the molecule. Sites of ligand-protein interaction can also be determined by analysis of crystal structure as determined by such techniques as nuclear magnetic resonance, crystallography or photoaffinity labeling. See, for example, de Vos et al., Science 255:306-312 (1992); Smith et al., J. Mol. Biol. 224:899-904, 1992; Wlodaver et al., FEBS Lett. 309:59-64 (1992). The identities of essential amino acids can also be inferred from analysis of homologies with related proteins.

Multiple amino acid substitutions can be made and tested using known methods of mutagenesis and screening, such as those disclosed by Reidhaar-Olson and Sauer, Science 241:53-57 (1988) or Bowie and Sauer, Proc. Natl. Acad. Sci. USA 86:2152-2156 (1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display, *e.g.*, Lowman et al., Biochem. 30:10832-10837 (1991); Ladner et al., U.S. Patent No. 5,223,409; Huse, WIPO Publication WO 92/06204) and region-directed mutagenesis, Derbyshire et al., Gene 46:145 (1986); Ner et al., DNA 7:127 (1988).

Mutagenesis methods as disclosed above can be combined with high-throughput screening methods to detect activity of cloned, mutagenized proteins in host cells. Preferred assays in this regard include cell proliferation assays and biosensor-based ligand-binding assays, which are described below. Mutagenized DNA molecules that encode active proteins or portions thereof (e.g., ligand-binding fragments) can be recovered from the host cells and rapidly sequenced using modern equipment. These methods allow the rapid determination of the importance of individual amino acid residues in a polypeptide of interest, and can be applied to polypeptides of unknown structure.

Using the methods discussed above, one of ordinary skill in the art can prepare a variety of polypeptides that are substantially identical to SEQ ID NOs: 2, 3, 4, 5, 9, 12, 17 or 18 or allelic variants thereof and retain the properties of the wild-type protein. As expressed and claimed herein the language, "a polypeptide as defined by SEQ ID NOs: 2, 3, 4, 5, 9, 12, 17 or 18" includes all allelic variants and species orthologs of the polypeptide.

Another embodiment of the present invention provides for a peptide or polypeptide comprising an epitope-bearing portion of a polypeptide of the invention. The epitope of the this polypeptide portion is an immunogenic or antigenic epitope of a polypeptide of the invention. A region of a protein to which an antibody can bind is defined as an "antigenic epitope". See for instance, Geysen, H.M. et al., Proc. Natl. Acad Sci. USA 81:3998-4002 (1984).

As to the selection of peptides or polypeptides bearing an antigenic epitope (*i.e.*, that contain a region of a protein molecule to which an antibody can bind), it is well known in the art that relatively short synthetic peptides that mimic part of a protein sequence are routinely capable of eliciting an antiserum that reacts with the partially mimicked protein. See Sutcliffe, J.G. et al. Science 219:660-666 (1983). Peptides capable of eliciting protein-reactive sera are frequently represented in the primary sequence of a protein, can be characterized by a set of simple chemical rules, and are confined neither to immunodominant regions of intact proteins (*i.e.*, immunogenic epitopes) nor to the amino or carboxyl termini. Peptides that are extremely hydrophobic and those of six or fewer residues generally are ineffective at inducing antibodies that bind to the mimicked protein; longer soluble peptides, especially those containing proline residues, usually are effective.

Antigenic epitope-bearing peptides and polypeptides of the invention are therefore useful to raise antibodies, including monoclonal antibodies, that bind specifically to a polypeptide of the invention. Antigenic epitope-bearing peptides and polypeptides of the present invention contain a sequence of at least nine, preferably between 15 to about 30 amino acids contained within the amino acid sequence of a polypeptide of the invention. However, peptides or polypeptides comprising a larger portion of an amino acid sequence of the invention, containing from 30 to 50 amino acids, or any length up to and including the entire amino acid sequence of a polypeptide of the invention, also are useful for inducing antibodies that react with the protein. Preferably, the amino acid sequence of the epitope-bearing peptide is selected to provide substantial solubility in aqueous solvents (*i.e.,* the sequence includes relatively hydrophilic residues and hydrophobic residues are preferably avoided); and sequences containing proline residues are particularly preferred. All of the polypeptides shown in the sequence listing contain antigenic epitopes to be used according to the present invention.

Polynucleotides, generally a cDNA sequence, of the present invention encode the above-described polypeptides. A cDNA sequence which encodes a polypeptide of the present invention is comprised of a series of codons, each amino acid residue of the polypeptide being encoded by a codon and each codon being comprised of three nucleotides. The amino acid residues are encoded by their respective codons as follows.
Alanine (Ala) is encoded by GCA, GCC, GCG or GCT;
Cysteine (Cys) is encoded by TGC or TGT;
Aspartic acid (Asp) is encoded by GAC or GAT;
Glutamic acid (Glu) is encoded by GAA or GAG;
Phenylalanine (Phe) is encoded by TTC or TTT;
Glycine (Gly) is encoded by GGA, GGC, GGG or GGT;
Histidine (His) is encoded by CAC or CAT;
Isoleucine (Ile) is encoded by ATA, ATC or ATT;
Lysine (Lys) is encoded by AAA, or AAG;
Leucine (Leu) is encoded by TTA, TTG, CTA, CTC, CTG or CTT;
Methionine (Met) is encoded by ATG;
Asparagine (Asn) is encoded by AAC or AAT;
Proline (Pro) is encoded by CCA, CCC, CCG or CCT;
Glutamine (Gln) is encoded by CAA or CAG;
Arginine (Arg) is encoded by AGA, AGG, CGA, CGC, CGG or CGT;
Serine (Ser) is encoded by AGC, AGT, TCA, TCC, TCG or TCT;
Threonine (Thr) is encoded by ACA, ACC, ACG or ACT;
Valine (Val) is encoded by GTA, GTC, GTG or GTT;
Tryptophan (Trp) is encoded by TGG; and
Tyrosine (Tyr) is encoded by TAC or TAT.

It is to be recognized that according to the present invention, when a cDNA is claimed as described above, it is understood that what is claimed are both the sense strand, the anti-sense strand, and the DNA as double-stranded having both the sense and anti-sense strand annealed together by their respective hydrogen bonds. Also claimed is the messenger RNA (mRNA) which encodes the polypeptides of the present invention, and which mRNA is encoded by the above-described cDNA. A messenger RNA (mRNA) will encode a polypeptide using the same codons as those defined above, with the exception that each thymine nucleotide (T) is replaced by a uracil nucleotide (U).

The protein polypeptides of the present invention, including full-length proteins, protein fragments (e.g. receptor-binding fragments), and fusion polypeptides can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., Molecular Cloning: A Laboratory Manual, (2nd ed.) (Cold Spring Harbor Laboratory Press, Cold Spring Harbor, NY, 1989).

In general, a DNA sequence encoding a Ztgfβ-9 polypeptide is operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers.

To direct a Ztgfβ-9 polypeptide into the secretory pathway of a host cell, a secretory signal sequence (also known as a leader sequence, prepro sequence or pre sequence) is provided in the expression vector. The secretory signal sequence may be that of the protein, or may be derived from another secreted protein [*e.g*., the tissue plasminogen activator (t-PA)] leader sequence or synthesized *de novo.* The secretory signal sequence is joined to the *Ztgfβ-9* DNA sequence in the correct reading frame. Secretory signal sequences are commonly positioned 5' to the DNA sequence encoding the polypeptide of interest, although certain signal sequences may be positioned elsewhere in the DNA sequence of interest (see, *e.g.*, Welch et al., U.S. Patent No. 5,037,743; Holland et al., U.S. Patent No. 5,143,830).

Cultured mammalian cells are preferred hosts within the present invention. Methods for introducing exogenous DNA into mammalian host cells include calcium phosphate-mediated transfection, Wigler et al., Cell 14:725 (1978); Corsaro and Pearson, Somatic Cell Genetics 7:603 (1981): Graham and Van der Eb, Virology 52:456 (1973), electroporation, Neumann et al., EMBO J. 1:841-845 (1982), DEAE-dextran mediated transfection, Ausubel et al., eds., Current Protocols in Molecular Biology (John Wiley and Sons, Inc., NY, 1987), and liposome-mediated transfection (Hawley-Nelson et al., Focus 15:73 (1993); Ciccarone et al., Focus 15:80 (1993). The production of recombinant polypeptides in cultured mammalian cells is disclosed, for example, by Levinson *et al*., U.S. Patent No. 4,713,339; Hagen *et al.,* U.S. Patent No. 4,784,950; Palmiter *et al*., U.S. Patent No. 4,579,821; and Ringold, U.S. Patent No. 4,656,134. Suitable cultured mammalian cells include the COS-1 (ATCC No. CRL 1650), COS-7 (ATCC No. CRL 1651), BHK (ATCC No. CRL 1632), BHK 570 (ATCC No. CRL 10314), 293 [ATCC No. CRL 1573; Graham et al., J. Gen. Virol. 36:59-72 (1977)] and Chinese hamster ovary (e.g. CHO-K1; ATCC No. CCL 61) cell lines. Additional suitable cell lines are known in the art and available from public depositories such as the American Type Culture Collection, Rockville, Maryland. In general, strong transcription promoters are preferred, such as promoters from SV-40 or cytomegalovirus. See, e.g., U.S. Patent No. 4,956,288. Other suitable promoters include those from metallothionein genes (U.S. Patent Nos. 4,579,821 and 4,601,978) and the adenovirus major late promoter.

Other higher eukaryotic cells can also be used as hosts, including plant cells, insect cells and avian cells. The use of *Agrobacterium rhizogenes* as a vector for expressing genes in plant cells has been reviewed by Sinkar et al., J. Biosci. (Bangalore) 11:47 (1987). Transformation of insect cells and production of foreign polypeptides therein is disclosed by Guarino *et al*., U.S. Patent No. 5,162,222 and WIPO publication WO 94/06463. Insect cells can be infected with recombinant baculovirus, commonly derived from Autographa californica nuclear polyhedrosis virus (AcNPV). DNA encoding the Ztgfβ-9 polypeptide is inserted into the baculoviral genome in place of the AcNPV polyhedrin gene coding sequence by one of two methods. The first is the traditional method of homologous DNA recombination between wild-type AcNPV and a transfer vector containing the *Ztgfβ-9* cDNA flanked by AcNPV sequences. Suitable insect cells, *e.g.* SF9 cells, are infected with wild-type AcNPV and transfected with a transfer vector comprising a *Ztgfβ-9* polynucleotide operably linked to an AcNPV polyhedrin gene promoter, terminator, and flanking sequences. See, King, L.A. and Possee, R.D., The Baculovirus Expression System: A Laboratory Guide, (Chapman & Hall, London); O'Reilly, D.R. et al., Baculovirus Expression Vectors: A Laboratory Manual (Oxford University Press, New York, New York, 1994); and, Richardson, C. D., Ed., Baculovirus Expression Protocols. Methods in Molecular Biology, (Humana Press, Totowa, NJ 1995). Natural recombination within an insect cell will result in a recombinant baculovirus which contains *Ztgfβ-9* driven by the polyhedrin promoter. Recombinant viral stocks are made by methods commonly used in the art.

The second method of making recombinant baculovirus utilizes a transposon-based system described by Luckow, V.A, et al., J Virol 67:4566 (1993). This system is sold in the Bac-to-Bac kit (Life Technologies, Rockville, MD). This system utilizes a transfer vector, pFastBacl^{™} (Life Technologies) containing a Tn7 transposon to move the DNA encoding the Ztgfβ-9 polypeptide into a baculovirus genome maintained in *E. coli* as a large plasmid called a "bacmid." The pFastBacl^{™} transfer vector utilizes the AcNPV polyhedrin promoter to drive the expression of the gene of interest, in this case Ztgfβ-9. However, pFastBac1^{™} can be modified to a considerable degree. The polyhedrin promoter can be removed and substituted with the baculovirus basic protein promoter (also known as Pcor, p6.9 or MP promoter) which is expressed earlier in the baculovirus infection, and has been shown to be advantageous for expressing secreted proteins. See, Hill-Perkins, M.S. and Possee, R.D., J Gen Virol 71:971 (1990); Bonning, B.C. et al., J Gen Virol 75:1551 (1994); and, Chazenbalk, G.D., and Rapoport, B., J Biol Chem 270:1543 (1995). In such transfer vector constructs, a short or long version of the basic protein promoter can be used. Moreover, transfer vectors can be constructed which replace the native Ztgfβ-9 secretory signal sequences with secretory signal sequences derived from insect proteins. For example, a secretory signal sequence from Ecdysteroid Glucosyltransferase (EGT), honey bee Melittin (Invitrogen, Carlsbad, CA), or baculovirus gp67 (PharMingen, San Diego, CA) can be used in constructs to replace the native Ztgfβ-9 secretory signal sequence. In addition, transfer vectors can include an in-frame fusion with DNA encoding an epitope tag at the C- or N-terminus of the expressed Ztgfβ-9 polypeptide, for example, a Glu-Glu epitope tag, Grussenmeyer, T. et al., Proc Natl Acad Sci. 82:7952 (1985). Using a technique known in the art, a transfer vector containing *ztgfβ-9* is transformed into *E. coli,* and screened for bacmids which contain an interrupted lacZ gene indicative of recombinant baculovirus. The bacmid DNA containing the recombinant baculovirus genome is isolated, using common techniques, and used to transfect *Spodoptera frugiperda* cells, *e.g.* Sf9 cells. Recombinant virus that expresses Ztgfβ-9 is subsequently produced. Recombinant viral stocks are made by methods commonly used the art.

The recombinant virus is used to infect host cells, typically a cell line derived from the fall army worm, *Spodoptera frugiperda.* See, in general, Glick and Pasternak, Molecular Biotechnology: Principles and Applications of Recombinant DNA, ASM Press, Washington, D.C. (1994). Another suitable cell line is the High FiveO^{™} cell line (Invitrogen) derived from *Trichoplusia ni* (U.S. Patent #5,300,435). Commercially available serum-free media are used to grow and maintain the cells. Suitable media are Sf900 II^{™} (Life Technologies) or ESF 921^{™} (Expression Systems) for the Sf9 cells; and Excell0405^{™} (JRH Biosciences, Lenexa, KS) or Express FiveO^{™} (Life Technologies) for the T. ni cells. The cells are grown up from an inoculation density of approximately 2-5 x 10⁵ cells to a density of 1-2 x 10⁶ cells at which time a recombinant viral stock is added at a multiplicity of infection (MOI) of 0.1 to 10, more typically near 3. The recombinant virus-infected cells typically produce the recombinant Ztgfβ-9 polypeptide at 12-72 hours post-infection and secrete it with varying efficiency into the medium. The culture is usually harvested 48 hours post-infection. Centrifugation is used to separate the cells from the medium (supernatant). The supernatant containing the z*** polypeptide is filtered through micropore filters, usually 0.45 µm pore size. Procedures used are generally described in available laboratory manuals (King, L. A. and Possee, R.D., *ibid.;* O'Reilly, D.R, *et al., ibid.;* Richardson, C. D., *ibid.).* Subsequent purification of the Ztgfβ-9 polypeptide from the supernatant can be achieved using methods described herein.

Drug selection is generally used to select for cultured mammalian cells into which foreign DNA has been inserted. Such cells are commonly referred to as "transfectants". Cells that have been cultured in the presence of the selective agent and are able to pass the gene of interest to their progeny are referred to as "stable transfectants." A preferred selectable marker is a gene encoding resistance to the antibiotic neomycin. Selection is carried out in the presence of a neomycin-type drug, such as G-418 or the like. Selection systems may also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. A preferred amplifiable selectable marker is dihydrofolate reductase, which confers resistance to methotrexate. Other drug resistance genes (e.g. hygromycin resistance, multi-drug resistance, and puromycin acetyltransferase) can also be used.

Other higher eukaryotic cells can also be used as hosts, including insect cells, plant cells and avian cells. Transformation of insect cells and production of foreign polypeptides therein is disclosed by Guarino et al., U.S. Patent No. 5,162,222; Bang et al., U.S. Patent No. 4,775,624; and WIPO publication WO 94/06463. The use of *Agrobacterium rhizogenes* as a vector for expressing genes in plant cells has been reviewed by Sinkar et al., J. Biosci. (Bangalore) 11:47-58 (1987).

Fungal cells, including yeast cells, and particularly cells of the genus *Saccharomyces,* can also be used within the present invention, such as for producing protein fragments or polypeptide fusions. Methods for transforming yeast cells with exogenous DNA and producing recombinant polypeptides therefrom are disclosed by, for example, Kawasaki, U.S. Patent No. 4,599,311; Kawasaki et al., U.S. Patent No. 4,931,373; Brake, U.S. Patent No. 4,870,008; Welch et al., U.S. Patent No. 5,037,743; and Murray et al., U.S. Patent No. 4,845,075. Transformed cells are selected by phenotype determined by the selectable marker, commonly drug resistance or the ability to grow in the absence of a particular nutrient (e.g., leucine). A preferred vector system for use in yeast is the *POT1* vector system disclosed by Kawasaki et al., U.S. Patent No. 4,931,373, which allows transformed cells to be selected by growth in glucose-containing media. Suitable promoters and terminators for use in yeast include those from glycolytic enzyme genes (see, *e.g.,* Kawasaki, U.S. Patent No. 4,599,311; Kingsman et al., U.S. Patent No. 4,615,974; and Bitter, U.S. Patent No. 4,977,092) and alcohol dehydrogenase genes. See also U.S. Patents Nos. 4,990,446; 5,063,154; 5,139,936 and 4,661,454. Transformation systems for other yeasts, including *Hansenula polymorpha, Schizosaccharomyces* pombe, *Kluyveromyces lactis, Kluyveromyces fragilis, Ustilago maydis, Pichia pastoris, Pichia methanolica, Pichia guillermondii* and *Candida maltosa* are known in the art. See, for example, Gleeson et al., J. Gen. Microbiol. 132:3459-3465 (1986) and Cregg, U.S. Patent No. 4,882,279. Aspergillus cells may be utilized according to the methods of McKnight et al., U.S. Patent No. 4,935,349. Methods for transforming *Acremonium chrysogenum* are disclosed by Sumino et al., U.S. Patent No. 5,162,228. Methods for transforming Neurospora are disclosed by Lambowitz, U.S. Patent No. 4,486,533.

Prokaryotic host cells, including strains of the bacteria *Escherichia coli, Bacillus* and other genera are also useful host cells within the present invention. Techniques for transforming these hosts and expressing foreign DNA sequences cloned therein are well known in the art, see, *e.g.*, Sambrook *et al*., *ibid.*). When expressing a Ztgfβ-9 polypeptide in bacteria such as *E. coli,* the polypeptide may be retained in the cytoplasm, typically as insoluble granules, or may be directed to the periplasmic space by a bacterial secretion sequence. In the former case, the cells are lysed, and the granules are recovered and denatured using, for example, guanidine isothiocyanate or urea. The denatured polypeptide can then be refolded and dimerized by diluting the denaturant, such as by dialysis against a solution of urea and a combination of reduced and oxidized glutathione, followed by dialysis against a buffered saline solution. In the latter case, the polypeptide can be recovered from the periplasmic space in a soluble and functional form by disrupting the cells (by, for example, sonication or osmotic shock) to release the contents of the periplasmic space and recovering the protein, thereby obviating the need for denaturation and refolding.

Transformed or transfected host cells are cultured according to conventional procedures in a culture medium containing nutrients and other components required for the growth of the chosen host cells. A variety of suitable media, including defined media and complex media, are known in the art and generally include a carbon source, a nitrogen source, essential amino acids, vitamins and minerals. Media may also contain such components as growth factors or serum, as required. The growth medium will generally select for cells containing the exogenously added DNA by, for example, drug selection or deficiency in an essential nutrient which is complemented by the selectable marker carried on the expression vector or co-transfected into the host cell.

Within one aspect of the present invention, a novel protein is produced by a cultured cell, and the cell or the protein is used to screen for a receptor or receptors for the protein, including the natural receptor, as well as interacting proteins such as dimerization partners, agonists and antagonists of the natural ligand.

### PROTEIN ISOLATION:

Expressed recombinant polypeptides (or chimeric polypeptides) can be purified using fractionation and/or conventional purification methods and media. Ammonium sulfate precipitation and acid or chaotrope extraction may be used for fractionation of samples. Exemplary purification steps may include hydroxyapatite, size exclusion, FPLC and reverse-phase high performance liquid chromatography. Suitable anion exchange media include derivatized dextrans, agarose, cellulose, polyacrylamide, specialty silicas, and the like. PEI, DEAE, QAE and Q derivatives are preferred, with DEAE Fast-Flow Sepharose (Pharmacia, Piscataway, NJ) being particularly preferred. Exemplary chromatographic media include those media derivatized with phenyl, butyl, or octyl groups, such as Phenyl-Sepharose FF (Pharmacia), Toyopearl butyl 650 (Toso Haas, Montgomeryville, PA), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups and/or carbohydrate moieties. Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Methods for binding receptor polypeptides to support media are well known in the art. Selection of a particular method is a matter of routine design and is determined in part by the properties of the chosen support. See, for example, Affinity Chromatography: Principles & Methods (Pharmacia LKB Biotechnology, Uppsala, Sweden, 1988).

The polypeptides of the present invention can be isolated by exploitation of their *properties.* For example, immobilized metal ion adsorption (IMAC) chromatography can be used to purify histidine-rich proteins. Briefly, a gel is first charged with divalent metal ions to form a chelate [E. Sulkowski, Trends in Biochem. 3:1-7 (1985)]. Histidine-rich proteins will be adsorbed to this matrix with differing affinities, depending upon the metal ion used, and will be eluted by competitive elution, lowering the pH, or use of strong chelating agents. Other methods of purification include purification of glycosylated proteins by lectin affinity chromatography and ion exchange chromatography [Methods in Enzymol., Vol. 182:529-39, "Guide to Protein Purification", M. Deutscher, (ed.), (Acad. Press, San Diego, 1990). Alternatively, a fusion of the polypeptide of interest and an affinity tag (e.g., polyhistidine, maltose-binding protein, an immunoglobulin domain) may be constructed to facilitate purification. Furthermore, to facilitate purification of the secreted polypeptide, an amino or carboxyl-terminal extension, such as a polyhistidine tag, substance P, FLAG^{®} peptide [Hopp et al., Bio/Technology 6:1204-1210 (1988); available from Eastman Kodak Co., New Haven, CT), a Glu-Glu affinity tag [Grussenmeyer et al., Proc. Natl. Acad. Sci. USA 82:7952-4 (1985)], or another polypeptide or protein for which an antibody or other specific binding agent is available, can be fused to Ztgfβ to aid in purification.

Mice engineered to express the Ztgfβ-9 gene, referred to as "transgenic mice" and mice that exhibit a complete absence of Ztgfβ-9 gene function, referred to as "knockout mice", may also be generated (Snouwaert et al., Science, 257:1083 (1992); Lowell, et al., Nature, 366:740-742 (1993); Capecchi, M.R., Science, 244:1288-1292, (1989); Palmiter, R.D. et al., Annu. Rev. Genet., 20:465-499, (1986). For example, transgenic mice that over-express Ztgfβ-9 either ubiquitously or under a tissue-specific or tissue-restricted promoter can be used to ask whether overexpression causes a phenotype. For example, overexpression of a wild-type Ztgfβ-9 polypeptide, polypeptide fragment or a mutant thereof may alter normal cellular processes resulting in a phenotype that identifies a tissue in which Ztgfβ-9 expression is functionally relevant and may indicate a therapeutic target for the Ztgfβ-9 protein, gene, its agonists or antagonists. Moreover, such over-expression may result in a phenotype that shows similarity with human diseases. Similarly, knockout Ztgfβ-9 mice can be used to determine where Ztgfβ-9 is absolutely required in *vivo.* The phenotype of knockout mice is predictive of the in *vivo* effects of that of a Ztgfβ-9 antagonist. The human Ztgfβ-9 cDNA can be used to isolate murine Ztgfβ-9 mRNA, cDNA and genomic DNA, which are subsequently used to generate knockout or transgenic mice. These mice may be employed to study the *Ztgfβ-9* gene and the protein encoded thereby in an in *vivo* system, and can be used as *in vivo* models for corresponding human diseases. Moreover, transgenic mouse expression of Ztgfβ-9 antisense polynucleotides or ribozymes directed against *ztgfβ-*9 or single chain antibodies to Ztgfβ-9 can be used to further elucidate the biology of Ztgfβ-9.

### Uses

Northern blot analysis of the expression of Ztgfβ-9 reveals that Ztgfβ-9 is highly expressed in the brain and spinal cord. Therefore, Ztgfβ-9 may play a role in the maintenance of spinal cord involving either glial cells or neurons. This indicates that Ztgfβ-9 can be used to treat a variety of neurodegenerative diseases such as amyotrophic lateral sclerosis (ALS), Alzheimer's disease, Huntington's disease, Parkinson's disease and peripheral neuropathies, or demyelinating diseases including multiple sclerosis. The tissue specificity of Ztgfβ-9 expression suggests that Ztgfβ-9 may be a growth and/or maintenance factor in the spinal cord and brain which can be used to treat spinal cord, brain or peripheral nervous system injuries. Ztgfβ-9 can also be administered to someone to treat a viral infection.

The present invention also provides reagents with significant therapeutic value. The Ztgfβ-9 polypeptide (naturally occurring or recombinant), fragments thereof, antibodies and anti-idiotypic antibodies thereto, along with compounds identified as having binding affinity to the Ztgfβ-9 polypeptide, should be useful in the treatment of conditions associated with abnormal physiology or development, including abnormal proliferation, e.g., cancerous conditions, or degenerative conditions. For example, a disease or disorder associated with abnormal expression or abnormal signaling by a Ztgfβ-9 polypeptide should be a likely target for an agonist or antagonist of the Ztgfβ-9 polypeptide. In particular, Ztgfβ-9 can be used to treat inflammation. Inflammation is a result of an immune response to an infection or as an autoimmune response to a self-antigen.

Antibodies to the Ztgfβ-9 polypeptide can be purified and then administered to a patient. These reagents can be combined for therapeutic use with additional active or inert ingredients, *e.g.,* in pharmaceutically acceptable carriers or diluents along with physiologically innocuous stabilizers and excipients. These combinations can be sterile filtered and placed into dosage forms as by lyophilization in dosage vials or storage in stabilized aqueous preparations. This invention also contemplates use of antibodies, binding fragments thereof or single-chain antibodies of the antibodies including forms which are not complement binding.

The quantities of reagents necessary for effective therapy will depend upon many different factors, including means of administration, target site, physiological state of the patient, and other medications administered. Thus, treatment dosages should be titrated to optimize safety and efficacy. Typically, dosages used *in vitro* may provide useful guidance in the amounts useful for *in vivo* administration of these reagents. Animal testing of effective doses for treatment of particular disorders will provide further predictive indication of human dosage. Methods for administration include oral, intravenous, peritoneal, intramuscular, or transdermal administration. Pharmaceutically acceptable carriers will include water, saline or buffers to name just a few. Dosage ranges would ordinarily be expected from 1µg to 1000µg per kilogram of body weight per day. However, the doses may be higher or lower as can be determined by a medical doctor with ordinary skill in the art. For a complete discussion of drug formulations and dosage ranges *see* Remington's Pharmaceutical Sciences, 17th Ed., (Mack Publishing Co., Easton, Penn., 1990), and Goodman and Gilman's: The Pharmacological Bases of Therapeutics, 9th Ed. (Pergamon Press 1996).

### Nucleic Acid-based Therapeutic Treatment

If a mammal has a mutated or lacks a *Ztgfβ*-9 gene, the *Ztgfβ*-9 gene can be introduced into the cells of the mammal. In one embodiment, a gene encoding a Ztgfβ-9 polypeptide is introduced *in vivo* in a viral vector. Such vectors include an attenuated or defective DNA virus, such as but not limited to herpes simplex virus (HSV), papillomavirus, Epstein Barr virus (EBV), adenovirus, adeno-associated virus (AAV), SV40 and the like. Defective viruses , which entirely or almost entirely lack viral genes, are preferred. A defective virus is not infective after introduction into a cell. Use of defective viral vectors allows for administration to cells in a specific, localized area, without concern that the vector can infect other cells. Examples of particular vectors include, but are not limited to, a defective herpes virus 1 (HSV1) vector [Kaplitt et al., Molec. Cell. Neurosci.,2 :320-330 (1991)], an attenuated adenovirus vector, such as the vector described by Stratford-Perricaudet et al., J. Clin. Invest., 90 :626-630 (1992), and a defective adeno-associated virus vector [Samulski et al., J. Virol., 61:3096-3101 (1987); Samulski et al. J. Virol., 63:3822-3828 (1989)].

In another embodiment, the gene can be introduced in a retroviral vector, *e.g.,* as described in Anderson *et al.*, U.S. Patent No. 5,399,346; Mann et al., Cell, 33:153 (1983); Temin et al., U.S. Patent No. 4,650,764; Temin et al., U.S. Patent No. 4,980,289; Markowitz et al., J. Virol., 62:1120 (1988); Temin et al., U.S. Patent No. 5,124,263; International Patent Publication No. WO 95/07358, published March 16, 1995 by Dougherty et al.; and Blood, 82:845 (1993).

Alternatively, the vector can be introduced by lipofection *in vivo* using liposomes. Synthetic cationic lipids can be used to prepare liposomes for *in vivo* transfection of a gene encoding a marker [Felgner et al., Proc. Natl. Acad. Sci. USA, 84:7413-7417 (1987); see Mackey et al., Proc. Natl. Acad. Sci. USA, 85:8027-8031 (1988)]. The use of lipofection to introduce exogenous genes into specific organs *in vivo* has certain practical advantages. Molecular targeting of liposomes to specific cells represents one area of benefit. It is clear that directing transfection to particular cell types would be particularly advantageous in a tissue with cellular heterogeneity, such as the pancreas, liver, kidney, and brain. Lipids may be chemically coupled to other molecules for the purpose of targeting. Targeted peptides, *e.g.*, hormones or neurotransmitters, and proteins such as antibodies, or non-peptide molecules could be coupled to liposomes chemically.

It is possible to remove the cells from the body and introduce the vector as a naked DNA plasmid or by means of a viral vector and then re-implant the transformed cells into the body. Naked DNA vector for gene therapy can be introduced into the desired host cells by methods known in the art, *e.g.,* transfection, electroporation, microinjection, transduction, cell fusion, DEAE dextran, calcium phosphate precipitation, use of a gene gun or use of a DNA vector transporter [see, *e.g*., Wu et al., J. Biol. Chem., 267:963-967 (1992); Wu et al., J. Biol. Chem., 263:14621-14624 (1988)]. Techniques such as viral vector-mediated gene delivery of Ztgfβ-9 can be used to treat human diseases such as cancer, immune & autoimmune diseases, and diseases of the central and peripheral nervous system.

ztgfβ-9 polypeptides can also be used to prepare antibodies that specifically bind to Ztgfβ-9 polypeptides. These antibodies can then be used to manufacture anti-idiotypic antibodies. As used herein, the term "antibodies" includes polyclonal antibodies, monoclonal antibodies, antigen-binding fragments thereof such as F(ab')₂ and Fab fragments, and the like, including genetically engineered antibodies. Antibodies are defined to be specifically binding if they bind to a Ztgfβ-9 polypeptide with a Kₐ of greater than or equal to 10⁷/M and they do not substantially bind to a polypeptide of the prior art. The affinity of a monoclonal antibody can be readily determined by one of ordinary skill in the art, for example, by using Scatchard analysis.

Methods for preparing polyclonal and monoclonal antibodies are well known in the art (see for example, Sambrook et al., Molecular Cloning: A Laboratory Manual, (Second Edition) (Cold Spring Harbor, NY, 1989); and Hurrell, J. G. R., Ed., Monoclonal Hybridoma Antibodies: Techniques and Applications (CRC Press, Inc., Boca Raton, FL, 1982). Polyclonal antibodies can be generated by inoculating a variety of warm-blooded animals such as horses, cows, goats, sheep, dogs, chickens, rabbits, mice, hamsters, guinea pigs and rats with a Ztgfβ-9 polypeptide or a fragment thereof. The immunogenicity of a Ztgfβ-9 polypeptide may be increased through the use of an adjuvant, such as alum (aluminum hydroxide) or Freund's complete or incomplete adjuvant. Polypeptides useful for immunization also include fusion polypeptides, such as fusions of Ztgfβ-9 or a portion thereof with an immunoglobulin polypeptide or with maltose binding protein. The polypeptide immunogen may be a full-length molecule or a portion thereof. If the polypeptide portion is "hapten-like", such portion may be advantageously joined or linked to a macromolecular carrier (such as keyhole limpet hemocyanin (KLH), bovine serum albumin (BSA) or tetanus toxoid) for immunization. A variety of assays known to those skilled in the art can be utilized to detect antibodies which specifically bind to Ztgfβ-9 polypeptides. Exemplary assays are described in detail in Antibodies: A Laboratory Manual, Harlow and Lane (Eds.), (Cold Spring Harbor Laboratory Press, 1988). Representative examples of such assays include: concurrent immunoelectrophoresis, radio-immunoassays, radio-immunoprecipitations, enzyme-linked immunosorbent assays (ELISA), dot blot assays, inhibition or competition assays, and sandwich assays.

As used herein, the term "antibodies" includes polyclonal antibodies, affinity-purified polyclonal antibodies, monoclonal antibodies, and antigen-binding fragments, such as F(ab')₂ and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced. Human antibodies can be generated in mice engineered to contain the human immunoglobulin loci, Vaughan, et al. Nat. Biotech., 16:535-539 (1998).

Alternative techniques for generating or selecting antibodies useful herein include *in vitro* exposure of lymphocytes to ztgfβ-9 protein or peptide, and selection of antibody display libraries in phage or similar vectors (for instance, through use of immobilized or labeled Ztgfβ-9 protein or peptide). Genes encoding polypeptides having potential Ztgfβ-9 polypeptide binding domains can be obtained by screening random peptide libraries displayed on phage (phage display) or on bacteria, such as *E. coli.* Nucleotide sequences encoding the polypeptides can be obtained in a number of ways, such as through random mutagenesis and random polynucleotide synthesis. These random peptide display libraries can be used to screen for peptides which interact with a known target which can be a protein or polypeptide, such as a ligand or receptor, a biological or synthetic macromolecule, or organic or inorganic substances. Techniques for creating and screening such random peptide display libraries are known in the art (Ladner et al., US Patent NO. 5,223,409; Ladner et al., US Patent NO. 4,946,778; Ladner et al., US Patent NO. 5,403,484 and Ladner et al., US Patent NO. 5,571,698) and random peptide display libraries and kits for screening such libraries are available commercially, for instance from Clontech (Palo Alto, CA), Invitrogen Inc. (San Diego, CA), New England Biolabs, Inc. (Beverly, MA) and Pharmacia LKB Biotechnology Inc. (Piscataway, NJ). Random peptide display libraries can be screened using the Ztgfβ-9 sequences disclosed herein to identify proteins which bind to Ztgfβ-9. These "binding proteins" which interact with Ztgfβ-9 polypeptides can be used for tagging cells; for isolating homolog polypeptides by affinity purification; they can be directly or indirectly conjugated to drugs, toxins, radionuclides and the like. These binding proteins can also be used in analytical methods such as for screening expression libraries and neutralizing activity. The binding proteins can also be used for diagnostic assays for determining circulating levels of polypeptides; for detecting or quantitating soluble polypeptides as marker of underlying pathology or disease. These binding proteins can also act as Ztgfβ-9 "antagonists" to block Ztgfβ-9 binding and signal transduction *in vitro* and *in vivo.*

Antibodies can also be generated by gene therapy. The animal is administered the DNA or RNA which encodes Ztgfβ-9 or an immunogenic fragment thereof so that cells of the animals are transfected with the nucleic acid and express the protein which in turn elicits an immunogenic response. Antibodies which then are produced by the animal are isolated in the form of polyclonal or monoclonal antibodies. Antibodies to Ztgfβ-9 may be used for tagging cells that express the protein, for affinity purification, within diagnostic assays for determining circulating levels of soluble protein polypeptides, and as antagonists to block ligand binding and signal transduction *in vitro* and *in vivo.*

Radiation hybrid mapping is a somatic cell genetic technique developed for constructing high-resolution, contiguous maps of mammalian chromosomes [Cox et al., Science 250:245-250 (1990)]. Partial or full knowledge of a gene's sequence allows the designing of PCR primers suitable for use with chromosomal radiation hybrid mapping panels. Commercially available radiation hybrid mapping panels which cover the entire human genome, such as the Stanford G3 RH Panel and the GeneBridge 4 RH Panel (Research Genetics, Inc., Huntsville, AL), are available. These panels enable rapid, PCR based, chromosomal localizations and ordering of genes, sequence-tagged sites (STSs), and other nonpolymorphic- and polymorphic markers within a region of interest. This includes establishing directly proportional physical distances between newly discovered genes of interest and previously mapped markers. The precise knowledge of a gene's position can be useful in a number of ways including: 1) determining if a sequence is part of an existing contig and obtaining additional surrounding genetic sequences in various forms such as genomic YAC-, BAC- or phage genomic clones or cDNA clones, 2) providing a possible candidate gene for an inheritable disease which shows linkage to the same chromosomal region, and 3) for cross-referencing model organisms such as mouse which may be beneficial in helping to determine what function a particular gene might have.

The present invention also provides reagents which will find use in diagnostic applications. For example, the *Ztgfβ*-9 gene has been mapped on chromosome 13q11.2-q11. A *Ztgfβ*-9 nucleic acid probe could to used to check for abnormalities on chromosome 13. For example, a probe comprising Ztgfβ-9 DNA or RNA or a subsequence thereof can be used to determine if the Ztgfβ-9 gene is present on human chromosome 13q11.2-q11 or if a mutation has occurred. Detectable chromosomal aberrations at the *Ztgf*β-9 gene locus include but are not limited to aneuploidy, gene copy number changes, insertions, deletions, restriction site changes and rearrangements. Such aberrations can be detected using polynucleotides of the present invention by employing molecular genetic techniques, such as restriction fragment length polymorphism (RFLP) analysis, short tandem repeat (STR) analysis employing PCR techniques, and other genetic linkage analysis techniques known in the art. Human *Ztgfβ-*9 maps at the 13q11.2-q11 region. Mouse *Ztgfβ*-9 maps to mouse chromosome 14 framework markers d14mit64 and dmit82 located at 22.0 and 19.5 centimorgans, respectively. The 19.5 cm region appears to be syntenic with the human locus containing the gap junction genes *gja3* and *gjb2.* See Mignon, C. et al., Cytogenet. Cell Genet. 72: 185-186 (1996).

The invention is further illustrated by the following non-limiting examples.

### Example 1.

### Cloning of Ztgfβ-9

Human *ztgfβ-9* was isolated from an arrayed pituitary gland cDNA plasmid library by PCR screening using SEQ ID NOs: 6 and 7. Thermocycler conditions were as follows: one cycle at 94°C for 3 minutes, 35 cycles at 94°C for 30 seconds, 62°C for 20 seconds, 72°C for 30 seconds, one cycle at 72°C for 5 minutes, followed by 4° C hold. The reactions were gel electrophoresed to identify positive pools and, in this way the library was deconvoluted to a pool of positive clones. These were electroporated into *E. coli* DH10B cells and plated for colony hybridization. The colonies were transferred to Hybond N filters (Amersham) and probed for positive colonies. Positive clones were sequenced for full length Ztgfβ-9.

Sequence analysis and conceptual translation of the human *Ztgfβ-9* cDNA (SEQ ID NOs: 1 and 16) predicts a protein product that is 202 amino acid residues in length. This protein is homologous to two members of the IL-17 family, Zcyto7, International Application No. PCT/US98/08212, and IL-17. Ztgfβ-9 shares 27.8% amino acid identity with Zcyto7 and 20.6% identity with IL-17 as determined by the Clustal Method using Lasergene MegAlign software. See Higgins and Sharp, CABIOS 5:151 (1989). In particular, Ztgfβ-9 shares four conserved cysteines (amino acid residues 114, 119, 167, 169 in SEQ ID NO:2) with Zcyto7 and IL-17. These cysteines are predicted to be involved in forming a cysteine-knot-like protein fold that is related to the found in TGF-β proteins.

### Example 2

Northern Analysis *Ztgfβ-9*

Analysis of tissue distribution was performed by the northern blotting technique using 2 adult and 1 fetal human brain blots, Human Multiple Tissue and Master Dot Blots from Clontech (Palo Alto, Ca). A probe was obtained by PCR using SEQ ID NOs:6 and 7. in a cDNA pool. Thermocycler conditions were as follows: one cycle at 94°C for 3 minutes, 35 cycles at 94°C for 10 seconds, 66°C for 20 seconds, 72°C for 30 seconds, one cycle at 72°C for 5 minutes, followed by 4°C hold. The reaction mixture was electrophoresed on a preparative agarose gel and a 162 bp fragment was gel purified using commercially available gel purification reagents and protocol (QIAEX II Gel Extraction Kit; Qiagen, Inc., Santa Clarita, Ca). The purified DNA was radioactively labeled with ³²P using a commercially available kit (Rediprime DNA labeling system; Amersham Corp., Arlington Heights, IL).The probe was purified using a NUCTRAP push column(Stratagene Cloning Systems, La Jolla, CA) . EXPRESSHYB (Clonetech, Palo Alto, CA) solution was used for prehybridization and hybridization. The hybridization solution consisted of 8 mls EXPRESSHYB, 80µl Sheared Salmon Sperm DNA (10mg/ml,5 Prime-3 Prime, Boulder, CO), 48 µl Human Cot-1 DNA (1mg/ml,GibcoBRL) and 18µl of radiolabeled probe. Hybridization took place overnight at 50°C And the blots were then washed in 2X SSC,0.1%SDS at RT, then2X SSC,0.1% SDS at 60°C, followed by 0.1X SSC, 0.1% SDS wash at 60°C. The blots were exposed overnight and developed. A major transcript signal of was observed on MTN blots in brain and spinal cord.

Master Dot blot signals were strong in all brain tissues (adult and fetal), spinal cord, heart, skeletal muscle, stomach, pancreas, adrenal gland, salivary gland, liver, small intestine, bone marrow, thymus, spleen, lymph node, heart, thyroid, trachea, testis, ovary and placenta.

### Example 3

### Cloning of Murine Ztgfβ-9

Full length sequence was obtained from a clone isolated from an arrayed mouse testis cDNA/plasmid library. The library was screened by PCR using oligonucleotides SEQ NO:10 and SEQ ID NO:11. The library was deconvoluted down to a positive pool of 250 clones. *E.coli* DH10B cells (Gibco BRL) were transformed with this pool by electroporation. The transformed culture was titered and arrayed out to 96 wells at ^{~}20 cells/well. The cells were grown up in LBamp overnight at 37°C. An aliquot of the cells were pelleted and PCR was used to identify a positive pool. The remaining cells from a positive pool were plated and colonies screened by PCR to identify a positive clone. The clone was sequenced and contained the putative full length sequence of murine Ztgfβ-*9*. The sequence of murine Ztgfbeta-9 is defined by SEQ ID NOs : 8 and 9.

### Example 4

### Northern Analysis of Mouse Ztgfβ-9

Northern analysis was also carried out on Mouse MTN and Master Dot blots (Clontech) and a Mouse Embryo blot. A full-length murine Ztgfβ-9 cDNA clone (see cloning section) was restriction digested with ApaI and EcoRI following standard protocols. The reaction was gel electrophoresed and the ~686bp fragment was gel purified using the Qiaex II Gel Purification Kit (Qiagen, Valencia, CA). The cDNA was P32-labeled using the Rediprime II Labeling Kit (Amersham) and column purified using reagents and protocols described earlier. Hybridization, washing, and detection were carried out under conditions as described in Example 2. A band was observed in heart, brain, lung, liver, skeletal muscle, kidney and testis. The Master Dot blot had strong signals in thyroid, with fainter signals in most other tissues. Hybridization to the Mouse Embryo blot indicated that Ztgfβ-9 was expressed at all stages examined (embryonic days 7, 11, 15, and 17).

By quantitative RT-PCR, murine Ztgfβ-9 was found to be highly expressed in the HCL hypothalamic cell line, and at lower levels in the GT1-1 and GT1-7 hypothalamic cell lines and the undifferentiated P19 teratocarcinoma cell line. Using quantitative RT-PCR, murine *Ztgfβ-9* was detected in neurons of the hippocampal cerebellar and olfactory cortex, Purkinje cells and other neuronal populations were heavily labeled in brain sections. The endothelium of the choroid plexus was also heavily positive. In the spinal cord, labeling was confined to the gray matter and appeared to be uniformly found in dorsal and ventral horn neurons representing sensory and motor neurons. Strong expression was also observed in the dorsal root ganglia.

### Example 5

### Antibody Production

Polypeptides SEQ ID NOs: 13, 14 and 15 were synthesized and were injected into rabbits and polyclonal anti-sera was subsequently affinity purified by column chromatography using the cognate immunogen. Also fusion proteins between full-length human and mouse Ztgf-β fused to the C-terminus of the maltose-binding protein were expressed in *E. coli* and purified by affinity chromatography over an amylose resin. Purified proteins were injected into rabbits and polyclonal anti-sera was subsequently affinity purified by column chromatography using the cognate immunogen.

### Example 6

### Immunocytochemistry

Affinity purified polyclonal antibodies to human Ztgfβ-9 produced according to the procedure of Example 5 were validated on normal COS cells and COS cells transfected with a *Ztgfβ-9* mammalian cell expression construct. Immunocytochemistry performed with anti-Ztgfβ-9 antibodies demonstrated Ztgfβ-9 expression in monkey brain and spinal cord. The immunocytochemistry staining was intracytoplasmic and observed in many large neurons and Purkinje cells. Scattered epithelial cells in the human duodenum also showed positive staining.

### Example 7

### Mammalian cell protein production

Human Ztgfβ-9 protein, both with and without a C-terminal a Glu-Glu affinity tag [Grussenmeyer et al., Proc. Natl. Acad. Sci. USA 82:7952-4 (1985)], was expressed in BHK cells using an expression vector in which Ztgfβ-9 expression is driven by the CMV immediate early promoter, a consensus intron from the variable region of mouse immunoglobulin heavy chain locus, multiple restriction sites for insertion of coding sequences, a stop codon and a human growth hormone terminator. The plasmid also has an *E. coli* origin of replication, a mammalian selectable marker expression unit having an SV40 promoter, enhancer and origin of replication, a DHFR gene and the SV40 terminator and Kozac sequences at the 5' end of the open reading frame.

Following selection for stable cell transfectants, media isolated from pools was analyzed by western blot under reducing and non-reducing conditions using a Ztgfβ-9 antibody or an anti-EE epitope tag antibody. Human Ztgfβ-9 was found to migrate under reducing conditions at 29 kDa. Since the predicted molecular weight of the fully processed form of the protein is 20.31 kDa, this suggests that the protein is glycosylated at one or both of two potential glycosylation sites. Under non-reducing conditions, Ztgfβ-9 protein migrated as at 49 kDa species. These results indicate that human Ztgfβ-9 is capable of forming a disulfide cross-linked homodimer. However, co-expression of C-terminally EE tagged human Ztgfβ-9 and untagged human Zcyto7, followed by affinity purification using an anti-EE tag antibody resulted in co-purification of both proteins, suggesting that in addition to Ztgfβ-9 homodimers, Ztgfβ-9 and Zcyto7 can also dimerize. Interaction of Ztgfβ-9 and Zcyto7 did not appear to be due to interchain disulfide-bonding between the proteins. C-terminal EE tagged human Ztgfβ-9 expressed in BHK cells was also anti-EE affinity purified and its N-terminal sequence determined. The signal cleavage site was found to occur proceeding amino acid A23.

### Example 8

### Transgenic mice

The open reading frame encoding full-length murine Ztgfβ-9 was amplified by PCR so as to introduce an optimized initiation codon and introduced into a transgenic vector in which expression of Ztgfβ-9 was regulated by the metallothionein I promoter. The transgene insert was separated from plasmid backbone by NotI digestion and agarose gel purification, and fertilized ova from matings of B6C3F1Tac mice were microinjected and implanted into pseudopregnant females. Founders were identified by PCR on genomic tail DNA (DNAeasy 96 kit; Qiagen). Transgenic lines were initiated by breeding founders with C57BL/6Tac mice. Animal protocols used in this study were approved by the ZymoGenetics Institutional Animal Care and Use Committee. From 49 progeny born only 8% were found to be transgenic (compared to an average of 20°s observed for a variety of other cDNAs driven by the same promoter), suggesting that high expression of murine Ztgfβ-9 may be embryonic lethal. Consistent with this, of the four founders identified, all expressed only low levels of Ztgfβ-9 mRNA in the liver. A fifth founder died at birth and interestingly, this animal expressed very high levels of Ztgfβ-9 mRNA in the liver (8500 copies/cell). Histopathological analysis of this animal identified severe apoptosis of the thymus and complete devaculization of brown fat. The expressing males were bred with wild-type females. One founder was capable of germline transmission, however all transgenic progeny of this founder either died at birth or were runted and died soon after weaning. Analysis of these animals identified a variety of phenotypes, including severe thymic apoptosis, devaculization of brown fat, liver hepatitis, and low lymphocyte peripheral blood cell numbers. These results indicate that Ztgfβ-9, its agonists and antagonists, and antibodies to Ztgfβ-9 will be useful in regulating immune cells, adipogenesis, and liver cells.

### Example 9

### Chromosomal position

Human Ztgfβ-9 was mapped to chromosome 13q11.2 on two radiation hybrid panels. The mouse Ztgfb9 gene links to murine chromosome 14 framework markers d14mit64 and dmit82 located at 22.0 and 19.5 centimorgans, respectively.

### Example 10

### Inhibition of Adenovirus Growth by Ztgfβ-9

The human and murine Ztgfβ-9 coding region were cloned into the adenovirus shuttle vector and recombined to generated the recombinant adenoviral genome. Transfection of *Ztgfβ-9* adenoviral genomes into 293A cells resulted in very small viral plaques (1 or 2 plaques per transfection which is low). These plaques did not expand in size. Normally plaques expand greatly in size over a period of 1-2 days as the virus replicates. The small plaques were harvested and we tried to expand the virus by infecting 293A cells. Infected monolayers again exhibited very low numbers of plaques and the plaques were small in size. These plaques did not expand in size over time. After 2-3 rounds of attempting to amplify the virus, eventually we did obtain a rapidly growing virus population. The virus that results from this amplification still contains the Ztgfβ-9 sequences. However, infection of cell by these viruses did not result in protein production. The initial behavior of both the mouse and human *Ztgf*β-*9*-containing viruses is unlike we have seen with any other cDNA. Clearly, virus replication was inhibited.

## Claims

1. An antibody which specifically binds to a polypeptide comprising an amino acid sequence that is at least 90% identical to an amino acid sequence selected from the group consisting of SEQ ID NO:2, SEQ ID NO:3, SEQ ID NO:4, SEQ ID NO:5, SEQ ID NO:9, SEQ ID NO:12, SEQ ID NO: 13, SEQ ID NO: 14, SEQ ID NO:15, SEQ ID NO:17, SEQ ID NO:18, SEQ ID NO: 19, SEQ ID NO: 20, SEQ ID NO:21 and SEQ ID NO:22.

2. An anti-idiotypic antibody which binds to an antibody according to Claim 1.

3. An isolated polynucleotide which encodes a polypeptide, wherein said polypeptide comprises an amino acid sequence that is at least 90% identical to an amino acid sequence selected from the group consisting of SEQ ID NO:2, SEQ ID NO:3, SEQ ID NO:4, SEQ ID NO:5, SEQ ID NO:9, SEQ ID NO:12, SEQ ID NO: 13, SEQ ID NO: 14, SEQ ID NO:15, SEQ ID NO:17, SEQ ID NO:18, SEQ ID NO: 19, SEQ ID NO: 20, SEQ ID NO: 21 and SEQ ID NO:22.

4. An isolated polynucleotide according to Claim 3, wherein the polynucleotide encodes a polypeptide comprising an amino acid sequence selected from the group consisting of SEQ ID NO:2, SEQ ID NO:3, SEQ ID NO:4, SEQ ID NO:5, SEQ ID NO:9, SEQ ID NO:12, SEQ ID NO: 13, SEQ ID NO: 14, SEQ ID NO:15, SEQ ID NO:17, and SEQ ID NO:18, SEQ ID NO: 19, SEQ ID NO: 20, SEQ ID NO: 21 and SEQ ID NO:22.

5. An isolated polynucleotide according to Claim 3 or Claim 4, wherein the polynucleotide is selected from the group consisting of SEQ ID N0:1 and SEQ ID NO:9.

6. An isolated polypeptide comprising an amino acid sequence, wherein said amino acid sequence is at least 90% identical to an amino acid sequence selected from the group consisting of SEQ ID NO:2, SEQ ID NO:3, SEQ ID NO:4, SEQ ID NO:5, SEQ ID NO:9, SEQ ID NO:12, SEQ ID NO: 13, SEQ ID NO: 14, SEQ ID NO:15, SEQ ID NO:17, SEQ ID NO:18, SEQ ID NO: 19, SEQ ID NO: 20, SEQ ID NO:21 and SEQ ID NO:22.

7. An isolated polypeptide according to Claim 6, wherein said amino acid sequence is selected from the group consisting of SEQ ID NO:2, SEQ ID NO:3, SEQ ID NO:4, SEQ ID NO:5, SEQ ID NO:9, SEQ ID NO:12, SEQ ID NO: 13, SEQ ID NO: 14, SEQ ID NO:15, SEQ ID NO:17, SEQ ID NO:18, SEQ ID NO: 19, SEQ ID NO: 20, SEQ ID NO:21 and SEQ ID NO:22.

8. Use of an antibody according to Claim 1, an anti-idiotypic antibody according to Claim 2, a polynucleotide according to any of Claims 3-5 or a polypeptide according to Claim 6 or 7 for the manufacture of a medicament for treating cancerous or degenerative conditions.

9. Use of an antibody according to Claim 1, a polynucleotide according to any of Claims 3-5 or a polypeptide according to Claim 6 or 7 for the manufacture of a medicament for regulating immune cells or liver cells, or for regulating adipogenesis.

10. Use of a polynucleotide according to any of Claims 3-5 or a polypeptide according to Claim 6 or 7 for the manufacture of a medicament for regulating the proliferation, differentiation or apoptosis of neurons, glial cells, lymphocytes, hematopoietic cells or stromal cells.

11. Use of a polynucleotide according to any of Claims 3-5 or a polypeptide according to Claim 6 or 7 for the manufacture of a medicament for treating neurodegenerative disease, demyelinating disease, inflammation or viral infection, or for treating spinal cord, brain or peripheral nervous system injuries.

12. Use according to Claim 11, wherein said neurodegenerative disease is selected from the group consisting of amyotrophic lateral sclerosis, Alzheimer's Disease, Huntingdon's Disease, Parkinson's Disease and peripheral neuropathies.

13. Use according to Claim 11, wherein said demyelinating disease is multiple sclerosis.

14. Use according to Claim 11, for treating inflammation that is the result of an immune response to infection or an autoimmune response to a self-antigen.


SEQUENCE LISTING

<110> ZymoGenetics, Inc.
      1201 Eastlake Avenue East
      Seattle, Washington 98102
      United States of America

<120> Mammalian Transforming Growth Factor
   Beta - 9

<130> 98-54PC

<150> 09/154,817
<151> 1998-09-17

<160> 22

<170> FastSEQ for Windows Version 3.0

<210> 1
<211> 1819
<212> DNA
<213> Homo sapiens

<220>
<221> CDS
<222> (71)...(676)

<400> 1
cgggcgcggg gcgcaggcgg gctcctccgg cgcgtgcgga cgctgagcgt ggcctgtccc      60
tcaggtctgg atg ctg gta gcc ggc ttc ctg ctg gcg ctg ccg ccg agc      109
           Met Leu Val Ala Gly Phe Leu Leu Ala Leu Pro Pro Ser
            1               5                   10

tgg gcc gcg ggc gcc ccg agg gcg ggc agg cgc ccc gcg cgg ccg cgg      157
Trp Ala Ala Gly Ala Pro Arg Ala Gly Arg Arg Pro Ala Arg Pro Arg
    15                  20                  25

ggc tgc gcg gac cgg ccg gag gag cta ctg gag cag ctg tac ggg cgc      205
Gly Cys Ala Asp Arg Pro Glu Glu Leu Leu Glu Gln Leu Tyr Gly Arg
30                  35                  40                  45

ctg gcg gcc ggc gtg ctc agt gcc ttc cac cac acg ctg cag ctg ggg      253
Leu Ala Ala Gly Val Leu Ser Ala Phe His His Thr Leu Gln Leu Gly

                50                  55                  60

ccg cgt gag cag gcg cgc aac gcg agc tgc ccg gca ggg ggc agg ccc 301
Pro Arg Glu Gln Ala Arg Asn Ala Ser Cys Pro Ala Gly Gly Arg Pro
            65                  70                  75

gcc gac cgc cgc ttc cgg ccg ccc acc aac ctg cgc agc gtg tcg ccc 349
Ala Asp Arg Arg Phe Arg Pro Pro Thr Asn Leu Arg Ser Val Ser Pro
        80                  85                  90

tgg gcc tac aga atc tcc tac gac ccg gcg agg tac ccc agg tac ctg 397
Trp Ala Tyr Arg Ile Ser Tyr Asp Pro Ala Arg Tyr Pro Arg Tyr Leu
    95                  100                 105

cct gaa gcc tac tgc ctg tgc cgg ggc tgc ctg acc ggg ctg ttc ggc 445
Pro Glu Ala Tyr Cys Leu Cys Arg Gly Cys Leu Thr Gly Leu Phe Gly
110                 115                 120                 125

gag gag gac gtg cgc ttc cgc agc gcc cct gtc tac atg ccc acc gtc 493
Glu Glu Asp Val Arg Phe Arg Ser Ala Pro Val Tyr Met Pro Thr Val
                130                 135                 140

gtc ctg cgc cgc acc ccc gcc tgc gcc ggc ggc cgt tcc gtc tac acc 541
Val Leu Arg Arg Thr Pro Ala Cys Ala Gly Gly Arg Ser Val Tyr Thr
            145                 150                 155

gag gcc tac gtc acc atc ccc gtg ggc tgc acc tgc gtc ccc gag ccg 589
Glu Ala Tyr Val Thr Ile Pro Val Gly Cys Thr Cys Val Pro Glu Pro
        160                 165                 170

gag aag gac gca gac agc atc aac tcc agc atc gac aaa cag ggc gcc 637
Glu Lys Asp Ala Asp Ser Ile Asn Ser Ser Ile Asp Lys Gln Gly Ala
    175                 180                 185

aag ctc ctg ctg ggc ccc aac gac gcg ccc gct ggc ccc tgaggccggt 686
Lys Leu Leu Leu Gly Pro Asn Asp Ala Pro Ala Gly Pro
190                 195                 200

cctgccccgg gaggtctccc cggcccgcat cccgaggcgc ccaagctgga gccgcctgga 746
gggctcggtc ggcgacctct gaagagagtg caccgagcaa accaagtgcc ggagcaccag 806
cgccgccttt ccatggagac tcgtaagcag cttcatctga cacgggaatc cctggcttgc 866
ttttagctac aagcaagcag cgtggctgga agctgatggg aaacgacccg gcacgggcat 926
cctgtgtgcg gcccgcatgg agggtttgga aaagttcacg gaggctccct gaggagcctc 986
tcagatcggc tgctgcgggt gcagggcgtg actcaccgct gggtgcttgc caaagagata 1046
gggacgcata tgctttttaa agcaatctaa aaataataat aagtatagcg actatatacc 1106

tacttttaaa atcaactgtt ttgaatagag gcagagctat tttatattat caaatgagag 1166
ctactctgtt acatttctta acatataaac atcgtttttt acttcttctg gtagaatttt 1226
ttaaagcata attggaatcc ttggataaat tttgtagctg gtacactctg gcctgggtct 1286
ctgaattcag cctgtcaccg atggctgact gatgaaatgg acacgtctca tctgacccac 1346
tcttccttcc actgaaggtc ttcacgggcc tccaggtgga ccaaagggat gcacaggcgg 1406
ctcgcatgcc ccagggccag ctaagagttc caaagatctc agatttggtt ttagtcatga 1466
atacataaac agtctcaaac tcgcacaatt ttttccccct tttgaaagcc actggggcca 1526
atttgtggtt aagaggtggt gagataagaa gtggaacgtg acatctttgc cagttgtcag 1586
aagaatccaa gcaggtattg gcttagttgt aagggcttta ggatcaggcc gaatatgagg 1646
acaaagtggg ccacgttagc atctgcagag atcaatctgg aggcttctgt ttctgcattc 1706
tgccacgaga gctaggtcct tgatcttttc tttagattga aagtctgtct ctgaacacaa 1766
ttatttgtaa aagttagaag ttctttttta aatcattaaa agaggcttgc tga 1819

<210> 2
<211> 202
<212> PRT
<213> Homo sapiens

<400> 2
Met Leu Val Ala Gly Phe Leu Leu Ala Leu Pro Pro Ser Trp Ala Ala
1               5                   10                  15
Gly Ala Pro Arg Ala Gly Arg Arg Pro Ala Arg Pro Arg Gly Cys Ala
            20                  25                  30
Asp Arg Pro Glu Glu Leu Leu Glu Gln Leu Tyr Gly Arg Leu Ala Ala
        35                  40                  45
Gly Val Leu Ser Ala Phe His His Thr Leu Gln Leu Gly Pro Arg Glu
    50                  55                  60
Gln Ala Arg Asn Ala Ser Cys Pro Ala Gly Gly Arg Pro Ala Asp Arg
65                  70                  75                  80
Arg Phe Arg Pro Pro Thr Asn Leu Arg Ser Val Ser Pro Trp Ala Tyr
                85                  90                  95
Arg Ile Ser Tyr Asp Pro Ala Arg Tyr Pro Arg Tyr Leu Pro Glu Ala
            100                 105                 110
Tyr Cys Leu Cys Arg Gly Cys Leu Thr Gly Leu Phe Gly Glu Glu Asp
        115                 120                 125
Val Arg Phe Arg Ser Ala Pro Val Tyr Met Pro Thr Val Val Leu Arg
    130                 135                 140
Arg Thr Pro Ala Cys Ala Gly Gly Arg Ser Val Tyr Thr Glu Ala Tyr
145                 150                 155                 160
Val Thr Ile Pro Val Gly Cys Thr Cys Val Pro Glu Pro Glu Lys Asp
                165                 170                 175
Ala Asp Ser Ile Asn Ser Ser Ile Asp Lys Gln Gly Ala Lys Leu Leu
            180                 185                 190
Leu Gly Pro Asn Asp Ala Pro Ala Gly Pro
        195                 200

<210> 3
<211> 187
<212> PRT
<213> Homo sapiens

<400> 3
Ala Gly Ala Pro Arg Ala Gly Arg Arg Pro Ala Arg Pro Arg Gly Cys
1               5                   10                  15
Ala Asp Arg Pro Glu Glu Leu Leu Glu Gln Leu Tyr Gly Arg Leu Ala
            20                  25                  30
Ala Gly Val Leu Ser Ala Phe His His Thr Leu Gln Leu Gly Pro Arg
        35                  40                  45
Glu Gln Ala Arg Asn Ala Ser Cys Pro Ala Gly Gly Arg Pro Ala Asp
    50                  55                  60
Arg Arg Phe Arg Pro Pro Thr Asn Leu Arg Ser Val Ser Pro Trp Ala
65                  70                  75                  80
Tyr Arg Ile Ser Tyr Asp Pro Ala Arg Tyr Pro Arg Tyr Leu Pro Glu
                85                  90                  95
Ala Tyr Cys Leu Cys Arg Gly Cys Leu Thr Gly Leu Phe Gly Glu Glu
            100                 105                 110
Asp Val Arg Phe Arg Ser Ala Pro Val Tyr Met Pro Thr Val Val Leu
        115                 120                 125
Arg Arg Thr Pro Ala Cys Ala Gly Gly Arg Ser Val Tyr Thr Glu Ala
    130                 135                 140
Tyr Val Thr Ile Pro Val Gly Cys Thr Cys Val Pro Glu Pro Glu Lys
145                 150                 155                 160
Asp Ala Asp Ser Ile Asn Ser Ser Ile Asp Lys Gln Gly Ala Lys Leu
                165                 170                 175
Leu Leu Gly Pro Asn Asp Ala Pro Ala Gly Pro
            180                 185

<210> 4
<211> 186
<212> PRT
<213> Homo sapiens

<400> 4
Gly Ala Pro Arg Ala Gly Arg Arg Pro Ala Arg Pro Arg Gly Cys Ala
1               5                   10                  15
Asp Arg Pro Glu Glu Leu Leu Glu Gln Leu Tyr Gly Arg Leu Ala Ala
            20                  25                  30
Gly Val Leu Ser Ala Phe His His Thr Leu Gln Leu Gly Pro Arg Glu
        35                  40                  45
Gln Ala Arg Asn Ala Ser Cys Pro Ala Gly Gly Arg Pro Ala Asp Arg

50                  55                  60
Arg Phe Arg Pro Pro Thr Asn Leu Arg Ser Val Ser Pro Trp Ala Tyr
65                  70                  75                  80
Arg Ile Ser Tyr Asp Pro Ala Arg Tyr Pro Arg Tyr Leu Pro Glu Ala
                85                  90                  95
Tyr Cys Leu Cys Arg Gly Cys Leu Thr Gly Leu Phe Gly Glu Glu Asp
            100                 105                 110
Val Arg Phe Arg Ser Ala Pro Val Tyr Met Pro Thr Val Val Leu Arg
        115                 120                 125
Arg Thr Pro Ala Cys Ala Gly Gly Arg Ser Val Tyr Thr Glu Ala Tyr
    130                 135                 140
Val Thr Ile Pro Val Gly Cys Thr Cys Val Pro Glu Pro Glu Lys Asp
145                 150                 155                 160
Ala Asp Ser Ile Asn Ser Ser Ile Asp Lys Gln Gly Ala Lys Leu Leu
                165                 170                 175
Leu Gly Pro Asn Asp Ala Pro Ala Gly Pro
            180                 185

<210> 5
<211> 185
<212> PRT
<213> Homo sapiens

<400> 5
Ala Pro Arg Ala Gly Arg Arg Pro Ala Arg Pro Arg Gly Cys Ala Asp
1               5                   10                  15
Arg Pro Glu Glu Leu Leu Glu Gln Leu Tyr Gly Arg Leu Ala Ala Gly
            20                  25                  30
Val Leu Ser Ala Phe His His Thr Leu Gln Leu Gly Pro Arg Glu Gln
        35                  40                  45
Ala Arg Asn Ala Ser Cys Pro Ala Gly Gly Arg Pro Ala Asp Arg Arg
    50                  55                  60
Phe Arg Pro Pro Thr Asn Leu Arg Ser Val Ser Pro Trp Ala Tyr Arg
65                  70                  75                  80
Ile Ser Tyr Asp Pro Ala Arg Tyr Pro Arg Tyr Leu Pro Glu Ala Tyr
                85                  90                  95
Cys Leu Cys Arg Gly Cys Leu Thr Gly Leu Phe Gly Glu Glu Asp Val
            100                 105                 110
Arg Phe Arg Ser Ala Pro Val Tyr Met Pro Thr Val Val Leu Arg Arg
        115                 120                 125
Thr Pro Ala Cys Ala Gly Gly Arg Ser Val Tyr Thr Glu Ala Tyr Val
    130                 135                 140
Thr Ile Pro Val Gly Cys Thr Cys Val Pro Glu Pro Glu Lys Asp Ala
145                 150                 155                 160
Asp Ser Ile Asn Ser Ser Ile Asp Lys Gln Gly Ala Lys Leu Leu Leu

165                    170                    175
Gly Pro Asn Asp Ala Pro Ala Gly Pro
        180                    185

<210> 6
<211> 23
<212> DNA
<213> Homo sapiens

<400> 6
ccgggtcgta ggagattctg tag                                              23

<210> 7
<211> 22
<212> DNA
<213> Homo sapiens

<400> 7
gcgtgctcag tgccttccac ca                                               22

<210> 8
<211> 1221
<212> DNA
<213> Mus musculis

<220>
<221> CDS
<222> (79)...(693)

<400> 8
gggtgtcgcc cttatttact tcgcagaaga gccttcagcc cccctcctaa caagtctgga      60
aagcatcacg gcgacgcg atg ttg ggg aca ctg gtc tgg atg ctc gcg gtc       111
                    Met Leu Gly Thr Leu Val Trp Met Leu Ala Val
                     1               5                  10

ggc ttc ctg ctg gca ctg gcg ccg ggc cgc gcg gcg ggc gcg ctg agg       159
Gly Phe Leu Leu Ala Leu Ala Pro Gly Arg Ala Ala Gly Ala Leu Arg
            15                  20                  25

acc ggg agg cgc ccg gcg cgg ccg cgg gac tgc gcg gac cgg ccg gag       207
Thr Gly Arg Arg Pro Ala Arg Pro Arg Asp Cys Ala Asp Arg Pro Glu
        30                  35                  40

gag ctc ctg gag cag ctg tac ggg cgg ctg gcg gcc ggc gtg ctc agc       255
Glu Leu Leu Glu Gln Leu Tyr Gly Arg Leu Ala Ala Gly Val Leu Ser

45                    50                    55

gcc ttc cac cac acg ctg cag ctc ggg ccg cgc gag cag gcg cgc aat     303
Ala Phe His His Thr Leu Gln Leu Gly Pro Arg Glu Gln Ala Arg Asn
60                  65                  70                  75

gcc agc tgc ccg gcc ggg ggc agg gcc gcc gac cgc cgc ttc cgg cca     351
Ala Ser Cys Pro Ala Gly Gly Arg Ala Ala Asp Arg Arg Phe Arg Pro
                80                  85                  90

ccc acc aac ctg cgc agc gtg tcg ccc tgg gcg tac agg att tcc tac     399
Pro Thr Asn Leu Arg Ser Val Ser Pro Trp Ala Tyr Arg Ile Ser Tyr
            95                  100                 105

gac cct gct cgc ttt ccg agg tac ctg ccc gaa gcc tac tgc ctg tgc     447
Asp Pro Ala Arg Phe Pro Arg Tyr Leu Pro Glu Ala Tyr Cys Leu Cys
        110                 115                 120

cga ggc tgc ctg acc ggg ctc tac ggg gag gag gac ttc cgc ttt cgc     495
Arg Gly Cys Leu Thr Gly Leu Tyr Gly Glu Glu Asp Phe Arg Phe Arg
    125                 130                 135

agc aca ccc gtc ttc tct cca gcc gtg gtg ctg cgg cgc aca gcg gcc     543
Ser Thr Pro Val Phe Ser Pro Ala Val Val Leu Arg Arg Thr Ala Ala
140                 145                 150                 155

tgc gcg ggc ggc cgc tct gtg tac gcc gaa cac tac atc acc atc ccg     591
Cys Ala Gly Gly Arg Ser Val Tyr Ala Glu His Tyr Ile Thr Ile Pro
                160                 165                 170

gtg ggc tgc acc tgc gtg ccc gag ccg gac aag tcc gcg gac agt gcg     639
Val Gly Cys Thr Cys Val Pro Glu Pro Asp Lys Ser Ala Asp Ser Ala
            175                 180                 185

aac tcc agc atg gac aag ctg ctg ctg ggg ccc gcc gac agg cct gcg     687
Asn Ser Ser Met Asp Lys Leu Leu Leu Gly Pro Ala Asp Arg Pro Ala
        190                 195                 200

ggg cgc tgatgccggg gactgcccgc catggcccag cttcctgcat gcatcaggtc     743
Gly Arg
    205

ccctggccct gacaaaaccc accccatgat ccctggccgc tgcctaattt ttccaaaagg     803
acagctacat aagctttaaa tatatttttc aaagtagaca ctacatatct acaactattt     863
tgaatagtgg cagaaactat tttcatatta gtaatttaga gcaagcatgt tgtttttaaa     923

cttctttgat atacaagcac atcacacaca tcccgttttc ctctagtagg attcttgagt 983
gcataattgt agtgctcaga tgaacttcct tctgctgcac tgtgccctgt ccctgagtct 1043
ctcctgtggc ccaagcttac taaggtgata atgagtgctc cggatctggg cacctaaggt 1103
ctccaggtcc ctggagaggg agggatgtgg gggggctaga accaagcgcc cctttgttct 1163
ttagcttatg gatggtctta actttataaa gattaaagtt tttggtgtta ttctttca 1221

<210> 9
<211> 205
<212> PRT
<213> Mus musculis

<400> 9
Met Leu Gly Thr Leu Val Trp Met Leu Ala Val Gly Phe Leu Leu Ala
1               5                   10                  15
Leu Ala Pro Gly Arg Ala Ala Gly Ala Leu Arg Thr Gly Arg Arg Pro
            20                  25                  30
Ala Arg Pro Arg Asp Cys Ala Asp Arg Pro Glu Glu Leu Leu Glu Gln
        35                  40                  45
Leu Tyr Gly Arg Leu Ala Ala Gly Val Leu Ser Ala Phe His His Thr
    50                  55                  60
Leu Gln Leu Gly Pro Arg Glu Gln Ala Arg Asn Ala Ser Cys Pro Ala
65                  70                  75                  80
Gly Gly Arg Ala Ala Asp Arg Arg Phe Arg Pro Pro Thr Asn Leu Arg
                85                  90                  95
Ser Val Ser Pro Trp Ala Tyr Arg Ile Ser Tyr Asp Pro Ala Arg Phe
            100                 105                 110
Pro Arg Tyr Leu Pro Glu Ala Tyr Cys Leu Cys Arg Gly Cys Leu Thr
        115                 120                 125
Gly Leu Tyr Gly Glu Glu Asp Phe Arg Phe Arg Ser Thr Pro Val Phe
    130                 135                 140
Ser Pro Ala Val Val Leu Arg Arg Thr Ala Ala Cys Ala Gly Gly Arg
145                 150                 155                 160
Ser Val Tyr Ala Glu His Tyr Ile Thr Ile Pro Val Gly Cys Thr Cys
                165                 170                 175
Val Pro Glu Pro Asp Lys Ser Ala Asp Ser Ala Asn Ser Ser Met Asp
            180                 185                 190
Lys Leu Leu Leu Gly Pro Ala Asp Arg Pro Ala Gly Arg
        195                 200                 205

<210> 10
<211> 23
<212> DNA
<213> Homo sapiens

<400> 10

gatcatgggg tgggttttgt cag                                                23

<210> 11
<211> 22
<212> DNA
<213> Homo sapiens

<400> 11
gaggacttcc gctttcgcaa ca                                                 22

<210> 12
<211> 183
<212> PRT
<213> Mus musculis

<400> 12
Ala Gly Ala Leu Arg Thr Gly Arg Arg Pro Ala Arg Pro Arg Asp Cys
1               5                   10                  15
Ala Asp Arg Pro Glu Glu Leu Leu Glu Gln Leu Tyr Gly Arg Leu Ala
            20                  25                  30
Ala Gly Val Leu Ser Ala Phe His His Thr Leu Gln Leu Gly Pro Arg
        35                  40                  45
Glu Gln Ala Arg Asn Ala Ser Cys Pro Ala Gly Gly Arg Ala Ala Asp
    50                  55                  60
Arg Arg Phe Arg Pro Pro Thr Asn Leu Arg Ser Val Ser Pro Trp Ala
65                  70                  75                  80
Tyr Arg Ile Ser Tyr Asp Pro Ala Arg Phe Pro Arg Tyr Leu Pro Glu
                85                  90                  95
Ala Tyr Cys Leu Cys Arg Gly Cys Leu Thr Gly Leu Tyr Gly Glu Glu
            100                 105                 110
Asp Phe Arg Phe Arg Ser Thr Pro Val Phe Ser Pro Ala Val Val Leu
        115                 120                 125
Arg Arg Thr Ala Ala Cys Ala Gly Gly Arg Ser Val Tyr Ala Glu His
    130                 135                 140
Tyr Ile Thr Ile Pro Val Gly Cys Thr Cys Val Pro Glu Pro Asp Lys
145                 150                 155                 160
Ser Ala Asp Ser Ala Asn Ser Ser Met Asp Lys Leu Leu Leu Gly Pro
                165                 170                 175
Ala Asp Arg Pro Ala Gly Arg
            180

<210> 13
<211> 30
<212> PRT
<213> Homo sapiens

<400> 13
Gln Leu Gly Pro Arg Glu Gln Ala Arg Asn Ala Ser Cys Pro Ala Gly
1               5                   10                  15
Gly Arg Pro Ala Asp Arg Arg Phe Arg Pro Pro Thr Asn Leu
            20                  25                  30

<210> 14
<211> 21
<212> PRT
<213> Homo sapiens

<400> 14
Gly Glu Glu Asp Val Arg Phe Arg Ser Ala Pro Val Tyr Met Pro Thr
1               5                   10                  15
Val Val Leu Arg Cys
            20

<210> 15
<211> 34
<212> PRT
<213> Homo sapiens

<400> 15
Cys Val Pro Glu Pro Glu Lys Asp Ala Asp Ser Ile Asn Ser Ser Ile
1               5                   10                  15
Asp Lys Gln Gly Ala Lys Leu Leu Leu Gly Pro Asn Asp Ala Pro Ala
            20                  25                  30
Gly Pro

<210> 16
<211> 2361
<212> DNA
<213> Homo sapiens

<220>
<221> CDS
<222> (572)...(1202)

<400> 16
gaattcggca cgagggtcag ggaagtattc agtgctttgt tgtagagttg ttggatagag     60
gcacaggatc atttcatgtt gttgaggaga aaggagcaac agcctcctcc caccttatta    120
aaaatagaga tttaaaaaaa cctctaattt cctcgaagta cagaatctca agaggtagct    180
ctaaggagaa tccctctggg tttgagcgca ttcctcttcc agggggccta ttcttggact    240

gctttcctta atagagaaat ctctctgagc caaaatcggc ctcccccaat tccatcctgt 300
cggccccact tttctgctcc ggagacttcc aagccagtcc ccactcctcc ttcagccagt 360
cgggcccgca cccgcgcccg gcagggccag ccctctcctc ctcctgcgtg gcgcagcaca 420
ggccctgagc gcgcgacccc aggccctggg cgccccgccg catgctcgcg gctggaagcc 480
ccagtttgcg tggcccttcg ggttattccg ctcaagagcc gccgcgtcgc cccatctcgg 540
cgcgaatctg aaagcgcttt cgggggagaa g atg ttg ggg gca ctg gtc tgg 592
Met Leu Gly Ala Leu Val Trp
1               5

atg ctg gta gcc ggc ttc ctg ctg gcg ctg ccg ccg agc tgg gcc gcg 640
Met Leu Val Ala Gly Phe Leu Leu Ala Leu Pro Pro Ser Trp Ala Ala
        10                  15                  20

ggc gcc ccg agg gcg ggc agg cgc ccc gcg cgg ccg cgg ggc tgc gcg 688
Gly Ala Pro Arg Ala Gly Arg Arg Pro Ala Arg Pro Arg Gly Cys Ala
    25                  30                  35

gac cgg ccg gag gag cta ctg gag cag ctg tac ggg cgc ctg gcg gcc 736
Asp Arg Pro Glu Glu Leu Leu Glu Gln Leu Tyr Gly Arg Leu Ala Ala
40                  45                  50                  55

ggc gtg ctc agt gcc ttc cac cac acg ctg cag ctg ggg ccg cgt gag 784
Gly Val Leu Ser Ala Phe His His Thr Leu Gln Leu Gly Pro Arg Glu
                60                  65                  70

cag gcg cgc aac gcg agc tgc ccg gca ggg ggc agg ccc gcc gac cgc 832
Gln Ala Arg Asn Ala Ser Cys Pro Ala Gly Gly Arg Pro Ala Asp Arg
            75                  80                  85

cgc ttc cgg ccg ccc acc aac ctg cgc agc gtg tcg ccc tgg gcc tac 880
Arg Phe Arg Pro Pro Thr Asn Leu Arg Ser Val Ser Pro Trp Ala Tyr
        90                  95                  100

aga atc tcc tac gac ccg gcg agg tac ccc agg tac ctg cct gaa gcc 928
Arg Ile Ser Tyr Asp Pro Ala Arg Tyr Pro Arg Tyr Leu Pro Glu Ala
    105                 110                 115

tac tgc ctg tgc cgg ggc tgc ctg acc ggg ctg ttc ggc gag gag gac 976
Tyr Cys Leu Cys Arg Gly Cys Leu Thr Gly Leu Phe Gly Glu Glu Asp
120                 125                 130                 135

gtg cgc ttc cgc agc gcc cct gtc tac atg ccc acc gtc gtc ctg cgc 1024
Val Arg Phe Arg Ser Ala Pro Val Tyr Met Pro Thr Val Val Leu Arg
                140                 145                 150

cgc acc ccc gcc tgc gcc ggc ggc cgt tcc gtc tac acc gag gcc tac     1072
Arg Thr Pro Ala Cys Ala Gly Gly Arg Ser Val Tyr Thr Glu Ala Tyr
            155                 160                 165

gtc acc atc ccc gtg ggc tgc acc tgc gtc ccc gag ccg gag aag gac     1120
Val Thr Ile Pro Val Gly Cys Thr Cys Val Pro Glu Pro Glu Lys Asp
        170                 175                 180

gca gac agc atc aac tcc agc atc gac aaa cag ggc gcc aag ctc ctg     1168
Ala Asp Ser Ile Asn Ser Ser Ile Asp Lys Gln Gly Ala Lys Leu Leu
    185                 190                 195

ctg ggc ccc aac gac gcg ccc gct ggc ccc tga g gccggtcctg            1212
Leu Gly Pro Asn Asp Ala Pro Ala Gly Pro  *
200                 205

ccccgggagg tctccccggc ccgcatcccg aggcgcccaa gctggagccg cctggagggc   1272
tcggtcggcg acctctgaag agagtgcacc gagcaaacca agtgccggag caccagcgcc   1332
gcctttccat ggagactcgt aagcagcttc atctgacacg ggaatccctg gcttgctttt   1392
agctacaagc aagcagcgtg gctggaagct gatgggaaac gacccggcac gggcatcctg   1452
tgtgcggccc gcatggaggg tttggaaaag ttcacggagg ctccctgagg agcctctcag   1512
atcggctgct gcgggtgcag ggcgtgactc accgctgggt gcttgccaaa gagataggga   1572
cgcatatgct ttttaaagca atctaaaaat aataataagt atagcgacta tatacctact   1632
tttaaaatca actgttttga atagaggcag agctatttta tattatcaaa tgagagctac   1692
tctgttacat ttcttaacat ataaacatcg ttttttactt cttctggtag aattttttaa   1752
agcataattg gaatccttgg ataaattttg tagctggtac actctggcct gggtctctga   1812
attcagcctg tcaccgatgg ctgactgatg aaatggacac gtctcatctg acccactctt   1872
ccttccactg aaggtcttca cgggcctcca ggtggaccaa agggatgcac aggcggctcg   1932
catgccccag ggccagctaa gagttccaaa gatctcagat ttggttttag tcatgaatac   1992
ataaacagtc tcaaactcgc acaatttttt cccccttttg aaagccactg gggccaattt   2052
gtggttaaga ggtggtgaga taagaagtgg aacgtgacat ctttgccagt tgtcagaaga   2112
atccaagcag gtattggctt agttgtaagg gctttaggat caggccgaat atgaggacaa   2172
agtgggccac gttagcatct gcagagatca atctggaggc ttctgtttct gcattctgcc   2232
acgagagcta ggtccttgat cttttcttta gattgaaagt ctgtctctga acacaattat   2292
ttgtaaaagt tagaagttct tttttaaatc attaaaagag gcttgctgaa aaaaaaaaaa   2352
aaaaaaaaa                                                           2361

<210> 17
<211> 209
<212> PRT
<213> Homo sapiens

<400> 17
Met Leu Gly Ala Leu Val Trp Met Leu Val Ala Gly Phe Leu Leu Ala
1               5                   10                  15

Leu Pro Pro Ser Trp Ala Ala Gly Ala Pro Arg Ala Gly Arg Arg Pro
            20                  25                  30
Ala Arg Pro Arg Gly Cys Ala Asp Arg Pro Glu Glu Leu Leu Glu Gln
        35                  40                  45
Leu Tyr Gly Arg Leu Ala Ala Gly Val Leu Ser Ala Phe His His Thr
    50                  55                  60
Leu Gln Leu Gly Pro Arg Glu Gln Ala Arg Asn Ala Ser Cys Pro Ala
65                  70                  75                  80
Gly Gly Arg Pro Ala Asp Arg Arg Phe Arg Pro Pro Thr Asn Leu Arg
                85                  90                  95
Ser Val Ser Pro Trp Ala Tyr Arg Ile Ser Tyr Asp Pro Ala Arg Tyr
            100                 105                 110
Pro Arg Tyr Leu Pro Glu Ala Tyr Cys Leu Cys Arg Gly Cys Leu Thr
        115                 120                 125
Gly Leu Phe Gly Glu Glu Asp Val Arg Phe Arg Ser Ala Pro Val Tyr
    130                 135                 140
Met Pro Thr Val Val Leu Arg Arg Thr Pro Ala Cys Ala Gly Gly Arg
145                 150                 155                 160
Ser Val Tyr Thr Glu Ala Tyr Val Thr Ile Pro Val Gly Cys Thr Cys
                165                 170                 175
Val Pro Glu Pro Glu Lys Asp Ala Asp Ser Ile Asn Ser Ser Ile Asp
            180                 185                 190
Lys Gln Gly Ala Lys Leu Leu Leu Gly Pro Asn Asp Ala Pro Ala Gly
        195                 200                 205
Pro

<210> 18
<211> 187
<212> PRT
<213> Homo sapiens

<400> 18
Ala Gly Ala Pro Arg Ala Gly Arg Arg Pro Ala Arg Pro Arg Gly Cys
1               5                   10                  15
Ala Asp Arg Pro Glu Glu Leu Leu Glu Gln Leu Tyr Gly Arg Leu Ala
            20                  25                  30
Ala Gly Val Leu Ser Ala Phe His His Thr Leu Gln Leu Gly Pro Arg
        35                  40                  45
Glu Gln Ala Arg Asn Ala Ser Cys Pro Ala Gly Gly Arg Pro Ala Asp
    50                  55                  60
Arg Arg Phe Arg Pro Pro Thr Asn Leu Arg Ser Val Ser Pro Trp Ala
65                  70                  75                  80
Tyr Arg Ile Ser Tyr Asp Pro Ala Arg Tyr Pro Arg Tyr Leu Pro Glu
                85                  90                  95

Ala Tyr Cys Leu Cys Arg Gly Cys Leu Thr Gly Leu Phe Gly Glu Glu
            100                 105                 110
Asp Val Arg Phe Arg Ser Ala Pro Val Tyr Met Pro Thr Val Val Leu
        115                 120                 125
Arg Arg Thr Pro Ala Cys Ala Gly Gly Arg Ser Val Tyr Thr Glu Ala
    130                 135                 140
Tyr Val Thr Ile Pro Val Gly Cys Thr Cys Val Pro Glu Pro Glu Lys
145                 150                 155                 160
Asp Ala Asp Ser Ile Asn Ser Ser Ile Asp Lys Gln Gly Ala Lys Leu
                165                 170                 175
Leu Leu Gly Pro Asn Asp Ala Pro Ala Gly Pro
            180                 185

<210> 19
<211> 54
<212> PRT
<213> Homo sapiens

<400> 19
Gln Leu Gly Pro Arg Glu Gln Ala Arg Asn Ala Ser Cys Pro Ala Gly
1               5                   10                  15
Gly Arg Pro Ala Asp Arg Arg Phe Arg Pro Pro Thr Asn Leu Arg Ser
            20                  25                  30
Val Ser Pro Trp Ala Tyr Arg Ile Ser Tyr Asp Pro Ala Arg Tyr Pro
        35                  40                  45
Arg Tyr Leu Pro Glu Ala
    50

<210> 20
<211> 16
<212> PRT
<213> Homo sapiens

<400> 20
Arg Pro Ala Asp Arg Arg Phe Arg Pro Pro Thr Asn Leu Arg Ser Val
1               5                   10                  15

<210> 21
<211> 57
<212> PRT
<213> Homo sapiens

<400> 21
Arg Arg Thr Pro Ala Cys Ala Gly Gly Arg Ser Val Tyr Thr Glu Ala
1               5                   10                  15

Tyr Val Thr Ile Pro Val Gly Cys Thr Cys Val Pro Glu Pro Glu Lys
            20                  25                  30
Asp Ala Asp Ser Ile Asn Ser Ser Ile Asp Lys Gln Gly Ala Lys Leu
        35                  40                  45
Leu Leu Gly Pro Asn Asp Ala Pro Ala
    50                  55

<210> 22
<211> 14
<212> PRT
<213> Homo sapiens

<400> 22
Ser Tyr Asp Pro Ala Arg Tyr Pro Arg Tyr Leu Pro Glu Ala
1               5                   10

Ala Tyr Cys Leu Cys Arg Gly Cys Leu Thr Gly Leu Phe Gly Glu Glu
100 105 110
Asp Val Arg Phe Arg Ser Ala Pro Val Tyr Met Pro Thr Val Val Leu
115 120 125
Arg Arg Thr Pro Ala Cys Ala Gly Gly Arg Ser Val Tyr Thr Glu Ala
130 135 140
Tyr Val Thr Ile Pro Val Gly Cys Thr Cys Val Pro Glu Pro Glu Lys
145 150 155 160
Asp Ala Asp Ser Ile Asn Ser Ser Ile Asp Lys Gln Gly Ala Lys Leu
165 170 175
Leu Leu Gly Pro Asn Asp Ala Pro Ala Gly Pro
180 185

<210> 19
<211> 54
<212> PRT
<213> Homo sapiens

<400> 19
Gln Leu Gly Pro Arg Glu Gln Ala Arg Asn Ala Ser Cys Pro Ala Gly
1               5                   10                  15
Gly Arg Pro Ala Asp Arg Arg Phe Arg Pro Pro Thr Asn Leu Arg Ser
            20                  25                  30
Val Ser Pro Trp Ala Tyr Arg Ile Ser Tyr Asp Pro Ala Arg Tyr Pro
        35                  40                  45
Arg Tyr Leu Pro Glu Ala
    50

<210> 20
<211> 16
<212> PRT
<213> Homo sapiens

<400> 20
Arg Pro Ala Asp Arg Arg Phe Arg Pro Pro Thr Asn Leu Arg Ser Val
1               5                   10                  15

<210> 21
<211> 57
<212> PRT
<213> Homo sapiens

<400> 21
Arg Arg Thr Pro Ala Cys Ala Gly Gly Arg Ser Val Tyr Thr Glu Ala
1               5                   10                  15

Tyr Val Thr Ile Pro Val Gly Cys Thr Cys Val Pro Glu Pro Glu Lys
            20                  25                  30
Asp Ala Asp Ser Ile Asn Ser Ser Ile Asp Lys Gln Gly Ala Lys Leu
        35                  40                  45
Leu Leu Gly Pro Asn Asp Ala Pro Ala
    50                  55

<210> 22
<211> 14
<212> PRT
<213> Homo sapiens

<400> 22
Ser Tyr Asp Pro Ala Arg Tyr Pro Arg Tyr Leu Pro Glu Ala
1               5                   10